# EUROPEAN PATENT APPLICATION

(11) **EP 4 112 595 A1**
(43) Date of publication of application: **04.01.2023**
(21) Application number: 21761442.9
(22) Date of filing: 25.02.2021
(51) Int. Cl.: C07C 63/331, C07F 1/08, C07F 3/06, C07F 15/04, C07F 7/00, B01J 20/22

(54) **METAL-ORGANIC FRAMEWORK WITH CARBOXYLIC ACID ION HAVING TERPHENYL SKELETON AS LIGAND**

(30) Priority: 27.02.2020 JP 2020032043
(71) Applicant: Rikkyo Educational Corporation, Tokyo 171-8501 (JP); Nippon Soda Co., Ltd., Tokyo 100-8165 (JP)
(72) Inventor: MINOURA, Mao, Tokyo 171-8501 (JP); SUGAMATA, Koh, Tokyo 171-8501 (JP); IIHAMA, Teruyuki, Tokyo 100-8165 (JP)
(74) Representative: Mewburn Ellis LLP
(86) International application number: PCT/JP2021/007113
(87) International publication number: WO 2021/172433

(57) **Abstract**

An object of the present invention is to provide a novel metal-organic framework with a dicarboxylic acid having a terphenyl skeleton as an organic ligand and a gas storage method using such a metal-organic framework. A metal-organic framework comprising a carboxylate ion of formula (I) and a multivalent metal ion bonded to each other. (In formula (I), R¹ and R² each independently are a hydroxy group or an unsubstituted or substituted Cl-6 alkyl group. R¹⁰ and R¹¹ each independently are an unsubstituted or substituted C1-6 alkyl group. R²⁰ is an unsubstituted or substituted Cl-6 alkyl group. Provided that, as substituents on R¹, R², R³, R⁴, R⁵, R⁶, R⁷, R⁸, R¹⁰, R¹¹, and R²⁰, a carboxy group (COOH) and a carboxylate ion group (COO-) are excluded. In addition, R¹⁰ and R¹¹ are not a methyl group at the same time.)

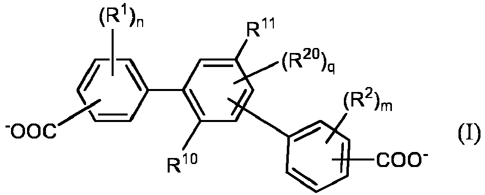

## Description

### Technical Field

The present invention relates to a novel metal-organic framework with a carboxylate ion having a terphenyl skeleton as a ligand and a method for storing a gas using such a metal-organic framework. This application claims priority to Japanese Patent Application No. 2020-032043 filed on February 27, 2020, the contents of which are incorporated herein.

### Background Art

Metal-organic frameworks (hereinafter sometimes referred to as a "MOFs") are substances in the form of a solid having a polymer structure having inner spaces (that is, pores) by combining metal ions with bridging organic ligands linking them, and they have attracted high interest as porous materials having functions such as gas storage and separation for the past dozen years or so. Much research on terephthalic acid used as an organic ligand has been conducted, and it has been known that MOF-5 obtained by a solvothermal method using Zn(NO₃)₂•6H₂O in DMF with terephthalic acid as a bridging organic ligand can store 7.1% by mass of hydrogen with respect to MOF-5 under the conditions of a temperature of 77 K and 4 MPa (see patent document 1 and non-patent documents 1 to 3).

Meanwhile, it has been known that a MOF having a surface area of 4000 m²/g obtained by heating zirconium chloride and terphenyl dicarboxylic acid in dimethylformamide can store a gas such as hydrogen, methane, or acetylene (see patent document 2).

In addition, it has been known that a MOF is obtained as a dark brown crystal by heating a dicarboxylic acid of the following formula and Fe₂CoO(CH₃COO)₆ or Fe₃O(CH₃COO)₆ in N-methyl pyrrolidone in the presence of acetic acid at 150°C for 24 hours and this MOF can store a gas such as hydrogen, methane, carbon dioxide, or nitrogen (see patent document 3) .

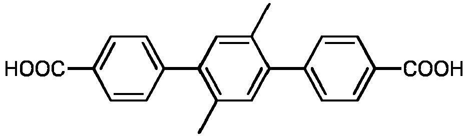

### Prior Art Documents

### Patent Documents

Patent Document 1: U.S. Patent Application Publication No. 2010-75123
Patent Document 2: International Publication No. WO 2009-133366
Patent Document 3: International Publication No. WO 2015-079229

### Non-patent Documents

Non-patent document 1: H. Li, M. Eddaudi, M. O'Keefe, O. M. Yaghi, Nature, 402, 276 (1999)
Non-patent document 2: M. Eddaudi, J. Kim, N. Rosi, D. Vodak, J. Wachter, M. O'Keefe, O. M. Yaghi, Science 2002, 295 (5554), 469.
Non-patent document 3: S. Kaye, A. Daily, O. M. Yaghi, J. Long, J. Am. Chem. Soc. 2007, 129 (46), 14176.

### Summary of the Invention

### Object to be Solved by the Invention

The structures of MOFs are known to change greatly depending on the metal species, ligand, and reaction conditions used. Regarding MOFs with a dicarboxylic acid having a terphenyl skeleton as an organic ligand, there are only a few examples of reports.

It is an object of the present invention to provide a novel MOF with a dicarboxylic acid having a terphenyl skeleton as an organic ligand and a gas storage method using such a MOF.

### Means to Solve the Object

The present inventors have studied diligently in order to solve the above problem, and as a result, found a novel MOF obtained by using a compound having a specific substituent at a specific site of a terphenyl skeleton as an organic ligand. In addition, the present inventors have found that this novel MOF has a high hydrogen storage capability and completed the present invention.

More specifically, the present invention is the following specified by the items shown below.
[1] A metal-organic framework comprising a carboxylate ion of formula (I) and a multivalent metal ion bonded to each other, 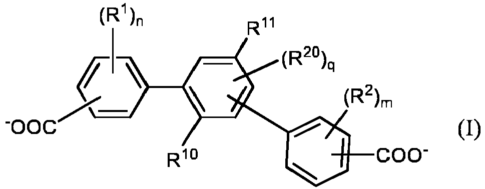 (in formula (I),
   R¹ and R² each independently are a hydroxy group, an unsubstituted or substituted C1-6 alkyl group, an unsubstituted or substituted C3-8 cycloalkyl group, an unsubstituted or substituted C6-10 aryl group, an unsubstituted or substituted three- to six-membered heterocyclyl group, an unsubstituted or substituted C1-6 alkoxy group, an unsubstituted or substituted C6-10 aryloxy group, an unsubstituted or substituted heteroaryloxy group, a halogeno group, an unsubstituted or substituted C1-6 alkylthio group, an unsubstituted or substituted C6-10 arylthio group, an unsubstituted or substituted heteroarylthio group, an unsubstituted or substituted C1-6 alkylsulfinyl group, an unsubstituted or substituted C6-10 arylsulfinyl group, an unsubstituted or substituted heteroarylsulfinyl group, an unsubstituted or substituted C1-6 alkylsulfonyl group, an unsubstituted or substituted C6-10 arylsulfonyl group, an unsubstituted or substituted heteroarylsulfonyl group, a cyano group, a nitro group, or a group of formula (II): 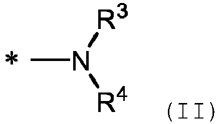
   (in formula (II), R³ and R⁴ each independently are a hydrogen atom, an unsubstituted or substituted C1-6 alkyl group, an unsubstituted or substituted C6-10 aryl group, an unsubstituted or substituted C1-6 alkylcarbonyl group, or an unsubstituted or substituted C6-10 arylcarbonyl group, and * is a bonding site),
   n is the number of R¹ and is 0, 1, 2, 3, or 4, and when n is 2 or more, a plurality of R¹s are the same or different from each other,
   m is the number of R² and is 0, 1, 2, 3, or 4, and when m is 2 or more, a plurality of R²s are the same or different from each other,
   R¹⁰ and R¹¹ each independently are an unsubstituted or substituted C1-6 alkyl group, an unsubstituted or substituted C3-8 cycloalkyl group, an unsubstituted or substituted C6-10 aryl group, an unsubstituted or substituted three- to six-membered heterocyclyl group, an unsubstituted or substituted C1-6 alkoxy group, an unsubstituted or substituted C6-10 aryloxy group, an unsubstituted or substituted heteroaryloxy group, a halogeno group, an unsubstituted or substituted C1-6 alkylthio group, an unsubstituted or substituted C6-10 arylthio group, an unsubstituted or substituted heteroarylthio group, an unsubstituted or substituted C1-6 alkylsulfinyl group, an unsubstituted or substituted C6-10 arylsulfinyl group, an unsubstituted or substituted heteroarylsulfinyl group, an unsubstituted or substituted C1-6 alkylsulfonyl group, an unsubstituted or substituted C6-10 arylsulfonyl group, an unsubstituted or substituted heteroarylsulfonyl group, a cyano group, a nitro group, or a group of formula (III),

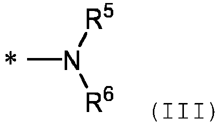

   (in formula (III), R⁵ and R⁶ each independently are a hydrogen atom, an unsubstituted or substituted C1-6 alkyl group, an unsubstituted or substituted C6-10 aryl group, an unsubstituted or substituted C1-6 alkylcarbonyl group, or an unsubstituted or substituted C6-10 arylcarbonyl group, and * is a bonding site),
   R²⁰ is an unsubstituted or substituted C1-6 alkyl group, an unsubstituted or substituted C3-8 cycloalkyl group, an unsubstituted or substituted C6-10 aryl group, an unsubstituted or substituted three- to six-membered heterocyclyl group, an unsubstituted or substituted C1-6 alkoxy group, an unsubstituted or substituted C6-10 aryloxy group, an unsubstituted or substituted heteroaryloxy group, a halogeno group, an unsubstituted or substituted C1-6 alkylthio group, an unsubstituted or substituted C6-10 arylthio group, an unsubstituted or substituted heteroarylthio group, an unsubstituted or substituted C1-6 alkylsulfinyl group, an unsubstituted or substituted C6-10 arylsulfinyl group, an unsubstituted or substituted heteroarylsulfinyl group, an unsubstituted or substituted C1-6 alkylsulfonyl group, an unsubstituted or substituted C6-10 arylsulfonyl group, an unsubstituted or substituted heteroarylsulfonyl group, a cyano group, a nitro group, or a group of formula (IV),

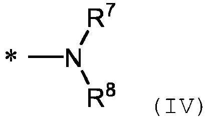

   (in formula (IV), R⁷ and R⁸ each independently are a hydrogen atom, an unsubstituted or substituted C1-6 alkyl group, an unsubstituted or substituted C6-10 aryl group, an unsubstituted or substituted C1-6 alkylcarbonyl group, or an unsubstituted or substituted C6-10 arylcarbonyl group, and * is a bonding site),
   q is the number of R²⁰ and is 0, 1, or 2, and when q is 2, R²⁰s are the same or different from each other,
   provided that, as substituents on R¹, R², R³, R⁴, R⁵, R⁶, R⁷, R⁸, R¹⁰, R¹¹, and R²⁰, a carboxy group (COOH) and a carboxylate ion group (COO-) are excluded, and in addition, R¹⁰ and R¹¹ are not a methyl group at the same time).
[2] The metal-organic framework according to [1], wherein the carboxylate ion of formula (I) is a carboxylate ion of formula (V), 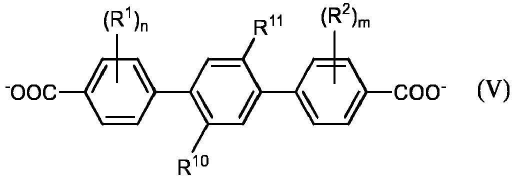 (wherein R¹, R², R¹⁰, R¹¹, n, and m have the same meaning as in formula (I)).
[3] The metal-organic framework according to [1] or [2], wherein the multivalent metal ion is an ion of at least one metal selected from the group consisting of metals of groups 2 to 13 of the periodic table of elements.
[4] The metal-organic framework according to any one of [1] to [3], wherein the multivalent metal ion is an ion of at least one metal selected from Zn, Fe, Co, Ni, Cu, Al, Zr, Ti, Cr, and Mg.
[5] The metal-organic framework according to any one of [1] to [4], further comprising an organic ligand other than the carboxylate ion of formula (I) as a constitution component.
[6] A gas storage method comprising a step of bringing a gas into contact with the metal-organic framework according to any one of [1] to [5] to adsorb or occlude the gas inside of the metal-organic framework.

### Effect of the Invention

The MOF of the present invention is novel, and the use of the MOF of the present invention makes it possible to store a gas such as hydrogen, carbon dioxide, or nitrogen.

### Mode of Carrying Out the Invention

A MOF of the present invention is a MOF comprising a multivalent metal ion and a dicarboxylic acid ion having a terphenyl skeleton bonded to each other.

The multivalent metal ion used in the present invention is not particularly limited as long as the multivalent metal ion is a di- or higher-valent metal ion, but is preferably an ion of at least one metal selected from the group consisting of metals of groups 2 to 13 of a periodic table of elements, furthermore, preferably an ion of at least one metal selected from Zn, Fe, Co, Ni, Cu, Al, Zr, Ti, Cr, and Mg, and, furthermore, preferably an ion of at least one metal selected from Co, Ni, Cu, Zr, Ti, Cr, and Zn, and these may be used alone or used by mixing of two or more thereof.

These multivalent metal ions are supplied in the form of various salts. As specific metal salts, zinc nitrate (Zn(NO₃)₂•xH₂O), titanium nitrate (Ti(NO₃)₄•xH₂O), cobalt nitrate (Co(NO₃)₂•xH₂O), iron (III) nitrate (Fe(NO₃)₃•xH₂O), iron (II) nitrate (Fe(NO₃)₂•xH₂O), nickel (II) nitrate (Ni(NO₃)₂•xH₂O), copper (II) nitrate (Cu(NO₃)₂•xH₂O); zinc chloride (ZnCl₂•xH₂O), titanium chloride (TiCl₄•xH₂O), zirconium chloride (ZrCl₄•xH₂O), cobalt chloride (COCl₂•xH₂O), iron (III) chloride (FeCl₃•xH₂O), iron (II) chloride (FeCl₂•xH₂O); zinc acetate (Zn(CH₃COO)₂•xH₂O), titanium acetate (Ti(CH₃COO)₄•xH₂O), zirconium acetate (Zr(CH₃COO)₄•xH₂O), cobalt acetate (Co(CH₃COO)₂•xH₂O), iron (III) acetate (Fe(CH₃COO)₃•xH₂O), iron (II) acetate (Fe(CH₃COO)₂•xH₂O); zinc sulfate (ZnSO₄•xH₂O), titanium sulfate (Ti(SO₄)₂•xH₂O), zirconium sulfate (Zr(SO₄)₂•xH₂O), cobalt sulfate (CoSO₄•xH₂O), iron (III) sulfate (Fe₂(SO₄)₃•xH₂O), iron (II) sulfate (FeSO₄•xH₂O); zinc hydroxide (Zn(OH)₂•xH₂O), titanium hydroxide (Ti(OH)₄•xH₂O), zirconium hydroxide (Zr(OH)₄•xH₂O), cobalt hydroxide (CO(OH)₂•xH₂O), iron (III) hydroxide (Fe(OH)₃•xH₂O), iron (II) hydroxide (Fe(OH)₂•xH₂O); zinc bromide (ZnBr₂•xH₂O), titanium bromide (TiBr₄•xH₂O), zirconium bromide (ZrBr₄•xH₂O), cobalt bromide (CoBr₂•xH₂O), iron (III) bromide (FeBr₃•xH₂O), iron (II) bromide (FeBr₂•xH₂O); zinc carbonate (ZnCO₃•xH₂O), cobalt carbonate (CoCO₃•XH₂O), iron (III) carbonate (Fe₂(CO₃)₃•xH₂O); zirconium chloride oxide (ZrOCl₂•xH₂O) and the like are exemplified. Provided that x is a number of 0 to 12. These may be used alone or used by mixing of two or more thereof.

The carboxylate ion having a terphenyl skeleton used in the present invention is represented by formula (I).

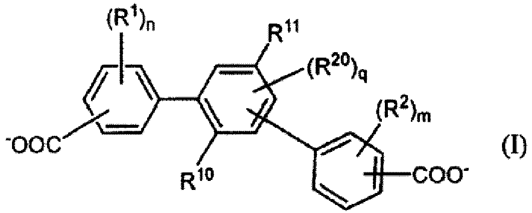

In formula (I), R¹ and R² each independently are a hydroxy group, an unsubstituted or substituted C1-6 alkyl group, an unsubstituted or substituted C3-8 cycloalkyl group, an unsubstituted or substituted C6-10 aryl group, an unsubstituted or substituted three- to six-membered heterocyclyl group, an unsubstituted or substituted C1-6 alkoxy group, an unsubstituted or substituted C6-10 aryloxy group, an unsubstituted or substituted heteroaryloxy group, a halogeno group, an unsubstituted or substituted C1-6 alkylthio group, an unsubstituted or substituted C6-10 arylthio group, an unsubstituted or substituted heteroarylthio group, an unsubstituted or substituted C1-6 alkylsulfinyl group, an unsubstituted or substituted C6-10 arylsulfinyl group, an unsubstituted or substituted heteroarylsulfinyl group, an unsubstituted or substituted C1-6 alkylsulfonyl group, an unsubstituted or substituted C6-10 arylsulfonyl group, an unsubstituted or substituted heteroarylsulfonyl group, a cyano group, a nitro group, or a group of formula (II).
n is the number of R¹ and is 0, 1, 2, 3, or 4, and when n is 2 or more, a plurality of R¹s are the same or different from each other.
m is the number of R² and is 0, 1, 2, 3, or 4, and when m is 2 or more, a plurality of R²s are the same or different from each other.

The C1-6 alkyl group as R¹ and R² may be linear or branched, and as the C1-6 alkyl group, a methyl group, an ethyl group, an n-propyl group, an n-butyl group, an n-pentyl group, an n-hexyl group, an i-propyl group, an i-butyl group, a s-butyl group, a t-butyl group, an i-pentyl group, a neopentyl group, a 2-methyl-n-butyl group, an i-hexyl group or the like is exemplified.

As the C3-8 cycloalkyl group as R¹ and R², a cyclopropyl group, a cyclobutyl group, a cyclopentyl group, a cyclohexyl group, a cubanyl group, or the like is exemplified.

The C6-10 aryl group as R¹ and R² may be either of a monocyclic ring and a polycyclic ring, and in the polycyclic aryl group, when at least one ring is an aromatic ring, the remaining ring may be any of a saturated alicyclic ring, an unsaturated alicyclic ring, or an aromatic ring. Specifically, a phenyl group, a naphthyl group, an azulenyl group, an indenyl group, an indanyl group, a tetralinyl group or the like is exemplified.

The three- to six-membered heterocyclyl group as R¹ and R² comprises, as a constituent atom of the ring, 1 to 4 heteroatoms selected from the group consisting of a nitrogen atom, an oxygen atom, and a sulfur atom. The heterocyclyl group may be either of a monocyclic ring and a polycyclic ring. In the polycyclic heterocyclyl group, when at least one ring is a heterocycle, the remaining ring may be any of a hydrocarbon ring of a saturated alicyclic ring, an unsaturated alicyclic ring, or an aromatic ring. As the three- to six-membered heterocyclyl group, a three- to six-membered saturated heterocyclyl group, a five- to six-membered heteroaryl group, a five- to six-membered partially unsaturated heterocyclyl group or the like is exemplified.

As the three- to six-membered saturated heterocyclyl group, an aziridinyl group, an epoxy group, a pyrrolidinyl group, a tetrahydrofuryl group, a thiazolidinyl group, a piperidyl group, a piperazinyl group, a morpholinyl group, a dioxolanyl group, a dioxanyl group or the like is exemplified.

As the five-membered heteroaryl group, a pyrrolyl group, a furyl group, a thienyl group, an imidazolyl group, a pyrazolyl group, an oxazolyl group, an isoxazolyl group, a thiazolyl group, an isothiazolyl group, a triazolyl group, an oxadiazolyl group, a thiadiazolyl group, a tetrazolyl group, an indonyl group, an isoindolinyl group, an indolizinyl group, a benzimidazolyl group, a carbazolyl group or the like is exemplified.

As the six-membered heteroaryl group, a pyridyl group, a pyrazyl group, a pyrimidyl group, a pyridazyl group, a triazyl group, a quinolinyl group, an isoquinolinyl group, a quinoxalinyl group, a cinnolinyl group, a quinazolyl group, a phthalazinyl group, an acridinyl group, a naphthazinyl group, a phenazinyl group or the like may be exemplified.

As the C1-6 alkoxy group as R¹ and R², a methoxy group, an ethoxy group, an n-propoxy group, an i-propoxy group, an n-butoxy group, a s-butoxy group, an i-butoxy group, and a t-butoxy group or the like is exemplified.

As the C6-10 aryloxy group as R¹ and R², a phenoxy group, a naphthyloxy group, an azulenyloxy group, an indenyloxy group, an indanyloxy group, a tetralinyloxy group or the like is exemplified.

As the heteroaryloxy group as R¹ and R², a furyloxy group, a thiazolyloxy group, a pyridyloxy group or the like is exemplified.

As the halogeno group as R¹ and R², a fluoro group, a chloro group, a bromo group, an iodo group or the like is exemplified.

As the C1-6 alkylthio group as R¹ and R², a methylthio group, an ethylthio group, an n-propylthio group, an i-propylthio group, an n-butylthio group, an i-butylthio group, an s-butylthio group, a t-butylthio group or the like is exemplified.

As the C6-10 arylthio group as R¹ and R², a phenylthio group, a naphthylthio group, an azulenylthio group, an indenylthio group, an indanylthio group, a tetralinylthio group or the like is exemplified.

As the heteroarylthio group as R¹ and R², a furylthio group, a thiazolylthio group, a pyridylthio group or the like is exemplified.

As the C1-6 alkylsulfinyl group as R¹ and R², a methylsulfinyl group, an ethylsulfinyl group, a t-butylsulfinyl group or the like is exemplified.

As the C6-10 arylsulfinyl group as R¹ and R², a phenylsulfinyl group, a naphthylsulfinyl group, an azulenylsulfinyl group, an indenylsulfinyl group, an indanylsulfinyl group, a tetralinylsulfinyl group or the like is exemplified.

As the heteroarylsulfinyl group as R¹ and R², a furylsulfinyl group, a thiazolylsulfinyl group, a pyridylsulfinyl group or the like is exemplified.

As the C1-6 alkylsulfonyl group as R¹ and R², a methylsulfonyl group, an ethylsulfonyl group, a t-butylsulfonyl group or the like is exemplified.

As the C6-10 arylsulfonyl group as R¹ and R², a phenylsulfonyl group, a naphthylsulfonyl group, an azulenylsulfonyl group, an indenylsulfonyl group, an indanylsulfonyl group, a tetralinylsulfonyl group or the like is exemplified.

As the heteroarylsulfonyl group as R¹ and R², a furylsulfonyl group, a thiazolylsulfonyl group, a pyridylsulfonyl group or the like is exemplified.

The group of formula (II) is shown below. 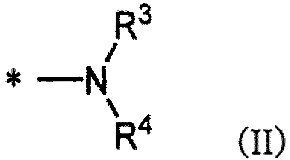 in formula (II), R³ and R⁴ each independently are a hydrogen atom, an unsubstituted or substituted C1-6 alkyl group, an unsubstituted or substituted C6-10 aryl group, an unsubstituted or substituted C1-6 alkylcarbonyl group, or an unsubstituted or substituted C6-10 arylcarbonyl group. * is a bonding site.

As the C1-6 alkyl group and the C6-10 aryl group as R³ and R⁴, those similar to those illustrated for the above R¹ and R² are exemplified.

As the C1-6 alkylcarbonyl group as R³ and R⁴, an acetyl group, an n-propionyl group, an isopropionyl group, an n-butyryl group, an isobutyryl group, a pivaloyl group, an n-pentanoyl group or the like is exemplified.

As the C6-10 arylcarbonyl group as R³ and R⁴, a benzoyl group, a 1-naphthylcarbonyl group, a 2-naphthylcarbonyl group or the like is exemplified.

As the group of formula (II), an amino group, a methylamino group, a dimethylamino group, an ethyl-i-propylamino group, an anilino group, a diphenylamino group, an acetylamino group, a benzoylamino group or the like is exemplified.

In formula (I), R¹⁰ and R¹¹ each independently are an unsubstituted or substituted C1-6 alkyl group, an unsubstituted or substituted C3-8 cycloalkyl group, an unsubstituted or substituted C6-10 aryl group, an unsubstituted or substituted three- to six-membered heterocyclyl group, an unsubstituted or substituted C1-6 alkoxy group, an unsubstituted or substituted C6-10 aryloxy group, an unsubstituted or substituted heteroaryloxy group, a halogeno group, an unsubstituted or substituted C1-6 alkylthio group, an unsubstituted or substituted C6-10 arylthio group, an unsubstituted or substituted heteroarylthio group, an unsubstituted or substituted C1-6 alkylsulfinyl group, an unsubstituted or substituted C6-10 arylsulfinyl group, an unsubstituted or substituted heteroarylsulfinyl group, an unsubstituted or substituted C1-6 alkylsulfonyl group, an unsubstituted or substituted C6-10 arylsulfonyl group, an unsubstituted or substituted heteroarylsulfonyl group, a cyano group, a nitro group, or a group of formula (III).

The C1-6 alkyl group as R¹⁰ and R¹¹ may be linear or branched, and a methyl group, an ethyl group, an n-propyl group, an n-butyl group, an n-pentyl group, an n-hexyl group, an i-propyl group, an i-butyl group, a s-butyl group, a t-butyl group, an i-pentyl group, a neopentyl group, a 2-methyl-n-butyl group, and an i-hexyl group or the like is exemplified.

As the C3-8 cycloalkyl group, the C6-10 aryl group, the three- to six-membered heterocyclyl group, the C1-6 alkoxy group, the C6-10 aryloxy group, the heteroaryloxy group, the halogeno group, the C1-6 alkylthio group, the C6-10 arylthio group, the heteroarylthio group, the C1-6 alkylsulfinyl group, the C6-10 arylsulfinyl group, the heteroarylsulfinyl group, the C1-6 alkylsulfonyl group, the C6-10 arylsulfonyl group, and the heteroarylsulfonyl group as R¹⁰ and R¹¹, those similar to those illustrated for R¹ and R² are exemplified.

The group of formula (III) is shown below. 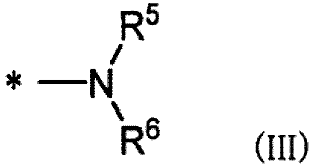 in formula (III), R⁵ and R⁶ each independently are a hydrogen atom, an unsubstituted or substituted C1-6 alkyl group, an unsubstituted or substituted C6-10 aryl group, an unsubstituted or substituted C1-6 alkylcarbonyl group, or an unsubstituted or substituted C6-10 arylcarbonyl group. * is a bonding site. In formula (III), as R⁵ and R⁶, those similar to those illustrated for R³ and R⁴ are exemplified, and as the group of formula (III), those similar to those illustrated for the group of formula (II) are exemplified.

In formula (I), R²⁰ is an unsubstituted or substituted C1-6 alkyl group, an unsubstituted or substituted C3-8 cycloalkyl group, an unsubstituted or substituted C6-10 aryl group, an unsubstituted or substituted three- to six-membered heterocyclyl group, an unsubstituted or substituted C1-6 alkoxy group, an unsubstituted or substituted C6-10 aryloxy group, an unsubstituted or substituted heteroaryloxy group, a halogeno group, an unsubstituted or substituted C1-6 alkylthio group, an unsubstituted or substituted C6-10 arylthio group, an unsubstituted or substituted heteroarylthio group, an unsubstituted or substituted C1-6 alkylsulfinyl group, an unsubstituted or substituted C6-10 arylsulfinyl group, an unsubstituted or substituted heteroarylsulfinyl group, an unsubstituted or substituted C1-6 alkylsulfonyl group, an unsubstituted or substituted C6-10 arylsulfonyl group, an unsubstituted or substituted heteroarylsulfonyl group, a cyano group, a nitro group, or a group of formula (IV).
q is the number of R²⁰ and is 0, 1, or 2, and when q is 2, R²⁰s are the same or different from each other.

As the C1-6 alkyl group, the C3-8 cycloalkyl group, the C6-10 aryl group, the three- to six-membered heterocyclyl group, the C1-6 alkoxy group, the C6-10 aryloxy group, the heteroaryloxy group, the halogeno group, the C1-6 alkylthio group, the C6-10 arylthio group, the heteroarylthio group, the C1-6 alkylsulfinyl group, the C6-10 arylsulfinyl group, the heteroarylsulfinyl group, the C1-6 alkylsulfonyl group, the C6-10 arylsulfonyl group, and the heteroarylsulfonyl group as R²⁰, those similar to those illustrated for R¹ and R² are exemplified.

The group of formula (IV) is shown below. 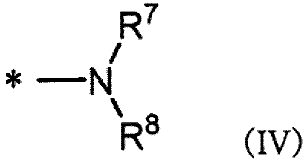 in formula (IV), R⁷ and R⁸ each independently are a hydrogen atom, an unsubstituted or substituted C1-6 alkyl group, an unsubstituted or substituted C6-10 aryl group, an unsubstituted or substituted C1-6 alkylcarbonyl group, or an unsubstituted or substituted C6-10 arylcarbonyl group. * is a bonding site. In formula (IV), as R⁷ and R⁸, those similar to those illustrated for R³ and R⁴ are exemplified, and as the group of formula (IV), those similar to those illustrated for the group of formula (II) are exemplified.

In the present invention, the term "unsubstituted" means only a group that is a mother nucleus. When only the name of the group that is a mother nucleus is mentioned, unless otherwise noted, it means that the group is "unsubstituted."

On the other hand, the term "substituted" means that any hydrogen atom in the group that is a mother nucleus is replaced by a group with the same or different structure as the mother nucleus. Therefore, "substituent" is a different group bonded to the group that is a mother nucleus. The number of substituents may be one or more. Two or more substituents are the same or different, and two or more substituents may bond to the same atom or different atoms.

"Substituent" is not particularly limited as long as the substituent is chemically allowed and has the effect of the present invention.

As specific examples of groups that can be "substituent," the following groups may be exemplified.

A C1-6 alkyl group such as a methyl group, an ethyl group, an n-propyl group, an i-propyl group, an n-butyl group, a s-butyl group, an i-butyl group, a t-butyl group, an n-pentyl group, or an n-hexyl group;
a C2-6 alkenyl group such as a vinyl group, a 1-propenyl group, a 2-propenyl group (allyl group), a propen-2-yl group, a 1-butenyl group, a 2-butenyl group, a 3-butenyl group, a 1-methyl-2-propenyl group, or a 2-methyl-2-propenyl group;
a C2-6 alkynyl group such as an ethynyl group, a 1-propynyl group, a 2-propynyl group (propargyl group), a 1-butynyl group, a 2-butynyl group, a 3-butynyl group, or a 1-methyl-2-propynyl group;
a C3-8 cycloalkyl group such as a cyclopropyl group, a cyclobutyl group, a cyclopentyl group, a cyclohexyl group, or a cubanyl group;
a C6-10 aryl group such as a phenyl group or a naphthyl group;
a C6-10 aryl C1-6 alkyl group such as a benzyl group or a phenethyl group;
a three- to six-membered heterocyclyl group;
a three- to six-membered heterocyclyl C1-6 alkyl group;
an oxo group;
a hydroxyl group;
a C1-6 alkoxy group such as a methoxy group, an ethoxy group, an n-propoxy group, an i-propoxy group, an n-butoxy group, a s-butoxy group, an i-butoxy group, or a t-butoxy group;
a C2-6 alkenyloxy group such as a vinyloxy group, an allyloxy group, a 1-propenyloxy group, a propen-2-yloxy group, a 3-butenyloxy group, or a 2-butenyloxy group;
a C2-6 alkynyloxy group such as an ethynyloxy group or a propargyloxy group;
a C6-10 aryloxy group such as a phenoxy group or a naphthoxy group;
a C6-10 aryl C1-6 alkoxy group such as a benzyloxy group or a phenethyloxy group;
a five- to six-membered heteroaryloxy group such as a thiazolyloxy group or a pyridyloxy group;
a five- to six-membered heteroaryl C1-6 alkyloxy group such as a thiazolylmethyloxy group or a pyridylmethyloxy group;
a formyl group;
a C1-6 alkylcarbonyl group such as an acetyl group or a propionyl group;
a formyloxy group;
a C1-6 alkylcarbonyloxy group such as an acetyloxy group or a propionyloxy group;
a C6-10 arylcarbonyl group such as a benzoyl group;
a C1-6 alkoxycarbonyl group such as a methoxycarbonyl group, an ethoxycarbonyl group, an n-propoxycarbonyl group, an i-propoxycarbonyl group, an n-butoxycarbonyl group, or a t-butoxycarbonyl group;
a C1-6 alkoxycarbonyloxy group such as a methoxycarbonyloxy group, an ethoxycarbonyloxy group, an n-propoxycarbonyloxy group, an i-propoxycarbonyloxy group, an n-butoxycarbonyloxy group, or a t-butoxycarbonyloxy group;
a halogeno group such as a fluoro group, a chloro group, a bromo group, or an iodo group;
a C1-6 haloalkyl group such as a fluoromethyl group, a difluoromethyl group, a trifluoromethyl group, a 2,2,2-trifluoroethyl group, a pentafluoroethyl group, a 3,3,3-trifluoropropyl group, a 2,2,3,3,3-pentafluoropropyl group, a perfluoropropyl group, a 2,2,2-trifluoro-1-trifluoromethylethyl group, a perfluoroisopropyl group, a 4-fluorobutyl group, a 2,2,3,3,4,4,4-heptafluorobutyl group, a perfluorobutyl group, a perfluoropentyl group, a perfluorohexyl group, a chloromethyl group, a bromomethyl group, a dichloromethyl group, a dibromomethyl group, a trichloromethyl group, a tribromomethyl group, a 1-chloroethyl group, a 2,2,2-trichloroethyl group, a 4-chlorobutyl group, a perchlorohexyl group, or a 2,4,6-trichlorohexyl group;
a C2-6 haloalkenyl group such as a 2-chloro-1-propenyl group or a 2-fluoro-1-butenyl group;
a C2-6 haloalkynyl group such as a 4,4-dichloro-1-butynyl group, a 4-fluoro-1-pentynyl group, or a 5-bromo-2-pentynyl group;
a C1-6 haloalkoxy group such as a trifluoromethoxy group, a 2-chloro-n-propoxy group, or a 2,3-dichlorobutoxy group;
a C2-6 haloalkenyloxy group such as a 2-chloropropenyloxy group or a 3-bromobutenyloxy group;
a C1-6 haloalkylcarbonyl group such as a chloroacetyl group, a trifluoroacetyl group, or a trichloroacetyl group;
an amino group;
a mono C1-6 alkyl-substituted amino group or a di-C1-6 alkyl-substituted amino group (in the latter case, C1-6 alkyl is the same or different from each other) such as a methylamino group a dimethylamino group, or a diethylamino group;
a C6-10 arylamino group such as an anilino group or a naphthylamino group;
a C6-10 aryl C1-6 alkylamino group such as a benzylamino group or a phenethylamino group;
a formylamino group;
a C1-6 alkylcarbonylamino group such as an acetylamino group, a propanoylamino group, a butyrylamino group, or an i-propylcarbonylamino group;
a C1-6 alkoxycarbonylamino group such as a methoxycarbonylamino group, an ethoxycarbonylamino group, an n-propoxycarbonylamino group, or an i-propoxycarbonylamino group;
a C1-6 alkylsulfoxyimino group such as a S,S-dimethylsulfoxyimino group;
an unsubstituted or substituted aminocarbonyl group, such as an aminocarbonyl group, a dimethylaminocarbonyl group, a phenylaminocarbonyl group, or an N-phenyl-N-methylaminocarbonyl group;
an imino C1-6 alkyl group such as an iminomethyl group, a 1-iminoethyl group, or a 1-imino-n-propyl group;
a substituted or unsubstituted N-hydroxyimino C1-6 alkyl group such as an N-hydroxy-iminomethyl group, a 1-(N-hydroxyimino)ethyl group, a 1-(N-hydroxyimino)propyl group, an N-methoxyiminomethyl group, or a 1-(N-methoxyimino)ethyl group;
a hydroxyimino group;
a C1-6 alkoxyimino group such as a methoxyimino group, an ethoxyimino group, an n-propoxyimino group, an i-propoxyimino group, or an n-butoxyimino group;
an aminocarbonyloxy group;
a mono C1-6 alkyl-substituted aminocarbonyloxy group or a di-C1-6 alkyl-substituted aminocarbonyloxy group (in the latter case, C1-6 alkyl is the same or different from each other) such as an ethylaminocarbonyloxy group or a dimethylaminocarbonyloxy group;
a thioxo group;
a thiol (mercapto) group;
a C1-6 alkylthio group such as a methylthio group, an ethylthio group, an n-propylthio group, an i-propylthio group, an n-butylthio group, an i-butylthio group, an s-butylthio group, and a t-butylthio group;
a C1-6 haloalkylthio group such as a trifluoromethylthio group, and a 2,2,2-trifluoroethylthio group;
a C6-10 arylthio group such as a phenylthio group or a naphthylthio group;
a five- to six-membered heteroarylthio group such as a thiazolylthio group or a pyridylthio group;
a C1-6 alkylsulfinyl group such as a methylsulfinyl group, an ethylsulfinyl group, or a t-butylsulfinyl group;
a C1-6 haloalkylsulfinyl group such as a trifluoromethylsulfinyl group or a 2,2,2-trifluoroethylsulfinyl group;
a C6-10 arylsulfinyl group such as a phenylsulfinyl group;
a five- to six-membered heteroarylsulfinyl group such as a thiazolylsulfinyl group or a pyridylsulfinyl group;
a C1-6 alkylsulfonyl group such as a methylsulfonyl group, an ethylsulfonyl group, or a t-butylsulfonyl group;
a C1-6 haloalkylsulfonyl group such as a trifluoromethylsulfonyl group or a 2,2,2-trifluoroethylsulfonyl group;
a C6-10 arylsulfonyl group such as a phenylsulfonyl group;
a five- to six-membered heteroarylsulfonyl group such as a thiazolylsulfonyl group or a pyridylsulfonyl group;
a sulfo group;
a C1-6 alkylsulfonyloxy group such as a methylsulfonyloxy group, an ethylsulfonyloxy group, or a t-butylsulfonyloxy group;
a C1-6 haloalkylsulfonyloxy group such as a trifluoromethylsulfonyloxy group or a 2,2,2-trifluoroethylsulfonyloxy group;
a tri-C1-6 alkyl-substituted silyl group such as a trimethylsilyl group, a triethylsilyl group, or a t-butyldimethylsilyl group;
a tri-C6-10 aryl-substituted silyl group such as a triphenylsilyl group;
a C2-C6 alkenyl C1-C6 dialkyl-substituted silyl group such as an allyldimethylsilyl group or a vinyldimethylsilyl group;
a C1-C6 alkyl di-C6-C10 aryl-substituted silyl group such as a t-butyldiphenylsilyl group or a diphenylmethylsilyl group;
a di-C1-C6 alkyl C6-C10 aryl-substituted silyl group such as a dimethylphenylsilyl group;
a (C6-C10 phenyl C1-C6 alkyl)di-C1-C6 alkylsilyl group such as a benzyldimethylsilyl group or a 3-phenylpropyldimethylsilyl group;
a C1-C6 alkyl C6-C10 aryl C2-C6 alkenylsilyl group such as a methylphenylvinylsilyl group;
a tri-C1-C6 alkoxy-substituted silyl group such as a trimethoxysilyl group or a triethoxysilyl group;
a di-C1-C6 alkyl-substituted silyl group such as a dimethylsilyl group or a diethylsilyl group;
a di-C1-C6 alkoxy-substituted silyl group such as a dimethoxysilyl group or a diethoxysilyl group;
a C1-C6 alkoxy di-C1-C6 alkyl-substituted silyl group such as a methoxydimethylsilyl group;
a C1-C6 alkoxy di-C6-C10 aryl-substituted silyl group such as a t-butoxydiphenylsilyl group;
a C1-C6 alkyl di-C1-C6 alkoxy-substituted silyl group such as a methyldimethoxysilyl group;
a cyano group; and
a nitro group.

The above "three- to six-membered heterocyclyl group" comprises, as a constituent atom of the ring, 1 to 4 heteroatoms selected from the group consisting of a nitrogen atom, an oxygen atom, and a sulfur atom. The heterocyclyl group may be either of a monocyclic ring and a polycyclic ring. In the polycyclic heterocyclyl group, when at least one ring is a heterocycle, the remaining ring may be any of a hydrocarbon ring of a saturated alicyclic ring, an unsaturated alicyclic ring, or an aromatic ring. As the "three- to six-membered heterocyclyl group", a three- to six-membered saturated heterocyclyl group, a five- to six-membered heteroaryl group, a five- to six-membered partially unsaturated heterocyclyl group or the like may be exemplified.

As the three- to six-membered saturated heterocyclyl group, an aziridinyl group, an epoxy group, a pyrrolidinyl group, a tetrahydrofuryl group, a thiazolidinyl group, a piperidyl group, a piperazinyl group, a morpholinyl group, a dioxolanyl group, a dioxanyl group or the like may be exemplified.

As the five-membered heteroaryl group, a pyrrolyl group, a furyl group, a thienyl group, an imidazolyl group, a pyrazolyl group, an oxazolyl group, an isoxazolyl group, a thiazolyl group, an isothiazolyl group, a triazolyl group, an oxadiazolyl group, a thiadiazolyl group, a tetrazolyl group, an indonyl group, an isoindolinyl group, an indolizinyl group, a benzimidazolyl group, a carbazolyl group or the like may be exemplified.

As the six-membered heteroaryl group, a pyridyl group, a pyrazinyl group, a pyrimidinyl group, a pyridazinyl group, a triazinyl group, a quinolinyl group, an isoquinolinyl group, a quinoxalinyl group, a cinnolinyl group, a quinazolyl group, a phthalazinyl group, an acridinyl group, a naphthazinyl group, a phenazinyl group or the like may be exemplified.

As the five- to six-membered partially unsaturated heterocyclyl group, an isoxazolinyl group, a pyrazolinyl group or the like may be exemplified.

As the three- to six-membered heterocyclyl 1-6 alkyl group, a glycidyl group, a 2-tetrahydrofurylmethyl group, a 2-pyrrolylmethyl group, a 2-imidazolylmethyl group, a 3-isoxazolylmethyl group, a 5-isoxazolylmethyl group, a 2-pyridylmethyl group, a 4-pyridylmethyl group, a 3-isoxazolinylmethyl group or the like may be exemplified.

The term "C1-6" or the like indicates that the number of carbon atoms in the group that is a mother nucleus is one to six or the like. This number of carbon atoms does not include the number of carbon atoms present in the substituent. For example, an ethoxybutyl group is classified into a C2 alkoxy C4 alkyl group since the group that is a mother nucleus is a butyl group and the substituent is an ethoxy group.

Provided that, in the case of using the term "C1-6 alkylcarbonyl" or "C6-10 arylcarbonyl", the number of carbon atoms does not include carbon in the carbonyl group.

As for these "substituents," any hydrogen atom in the substituent may be replaced by a group with a different structure.

Provided that, in formula (I), as substituents on R¹, R², R³, R⁴, R⁵, R⁶, R⁷, R⁸, R¹⁰, R¹¹, and R²⁰, a carboxy group (COOH) and a carboxylate ion group (COO-) are excluded. This means that, for example, in a case where R¹ is an alkyl group, a carboxyalkyl group is not regarded as a substituted alkyl group. In addition, R¹⁰ and R¹¹ are not a methyl group at the same time.

The carboxylate ion of formula (I) is not particularly limited as long as the carboxylate ion satisfies the condition of a chemical formula of formula (I), the substitution site of the carboxylate ion group, the substitution sites of R¹, R², and R²⁰, and the like may be arbitrarily selected, but, among them, a carboxylate ion of the following formula (V) is preferable.

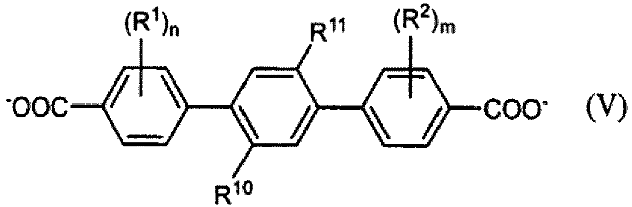

In formula (V), R¹, R², R¹⁰, R¹¹, n, and m have the same meanings as in formula (I).

Specifically, as the carboxylate ions of formula (V), compounds of the following formula are exemplified.

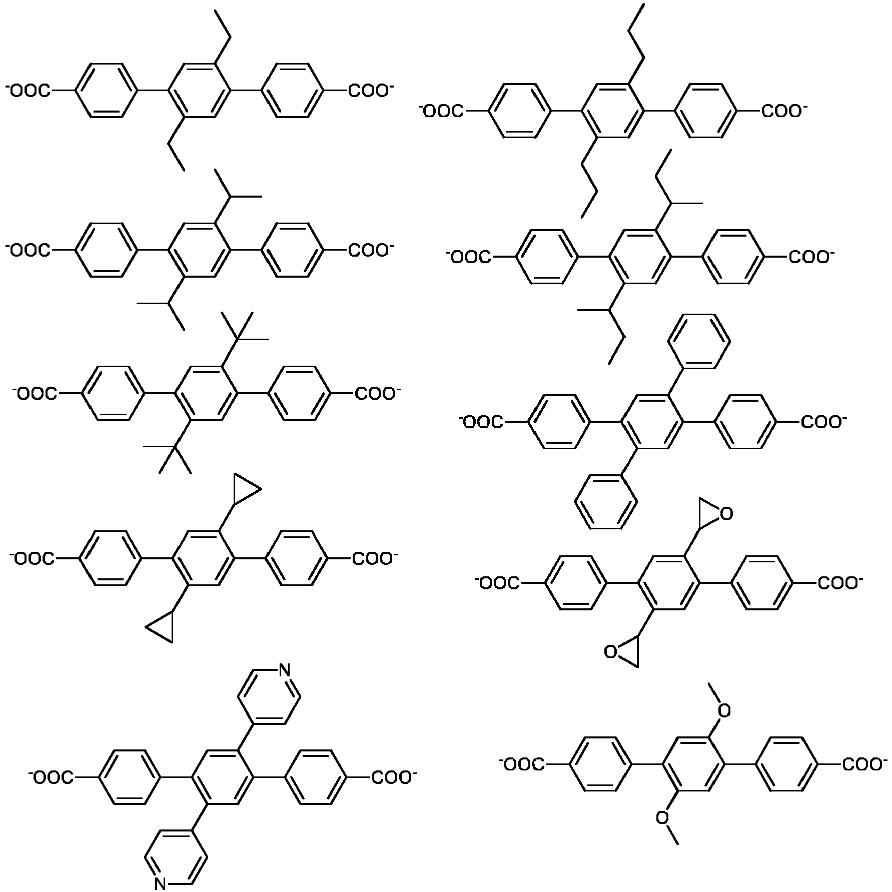

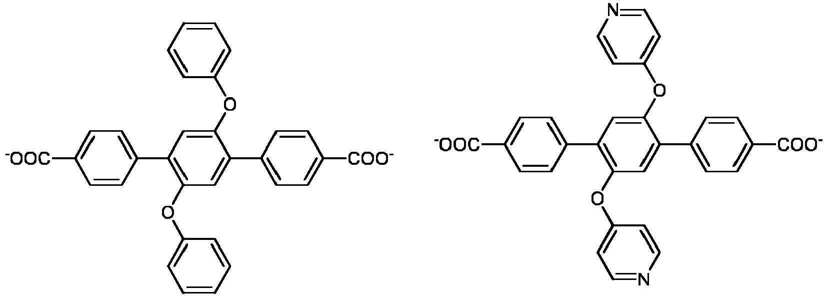

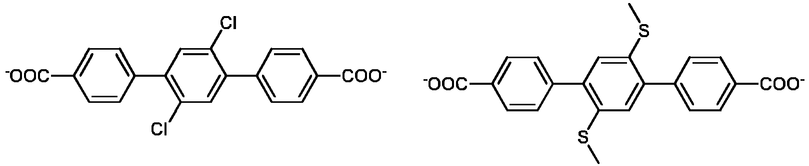

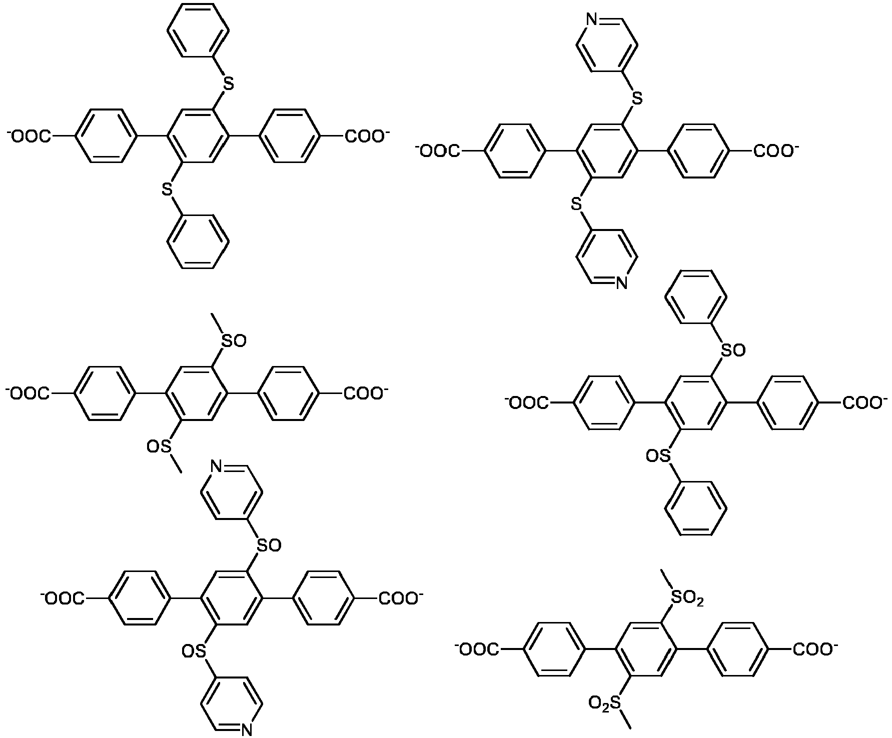

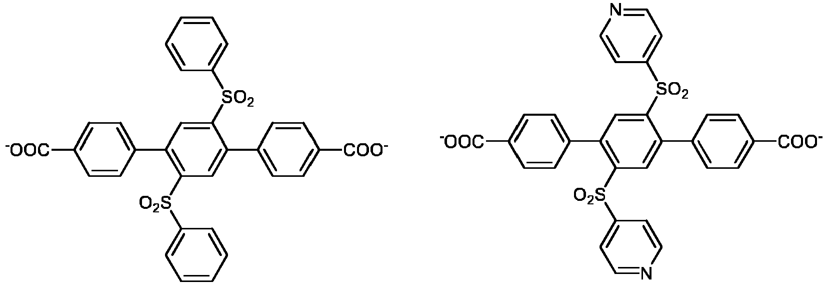

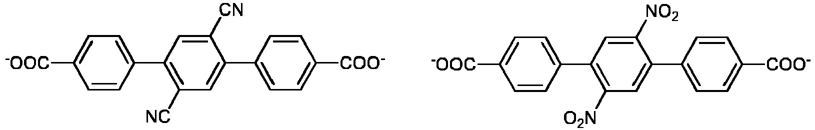

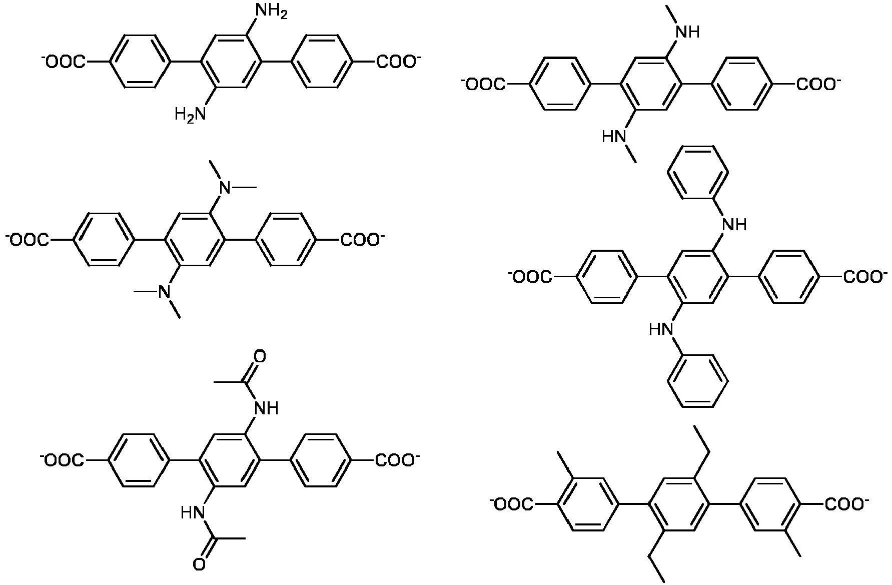

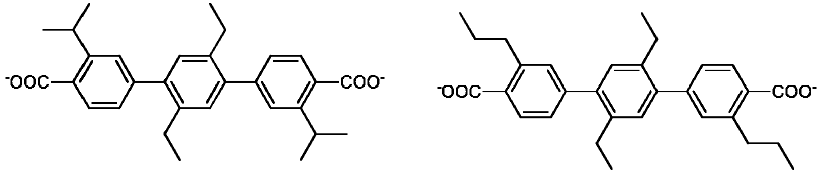

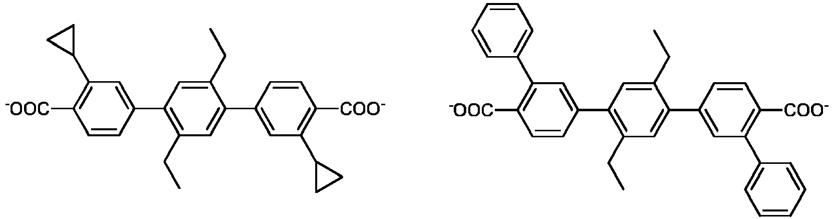

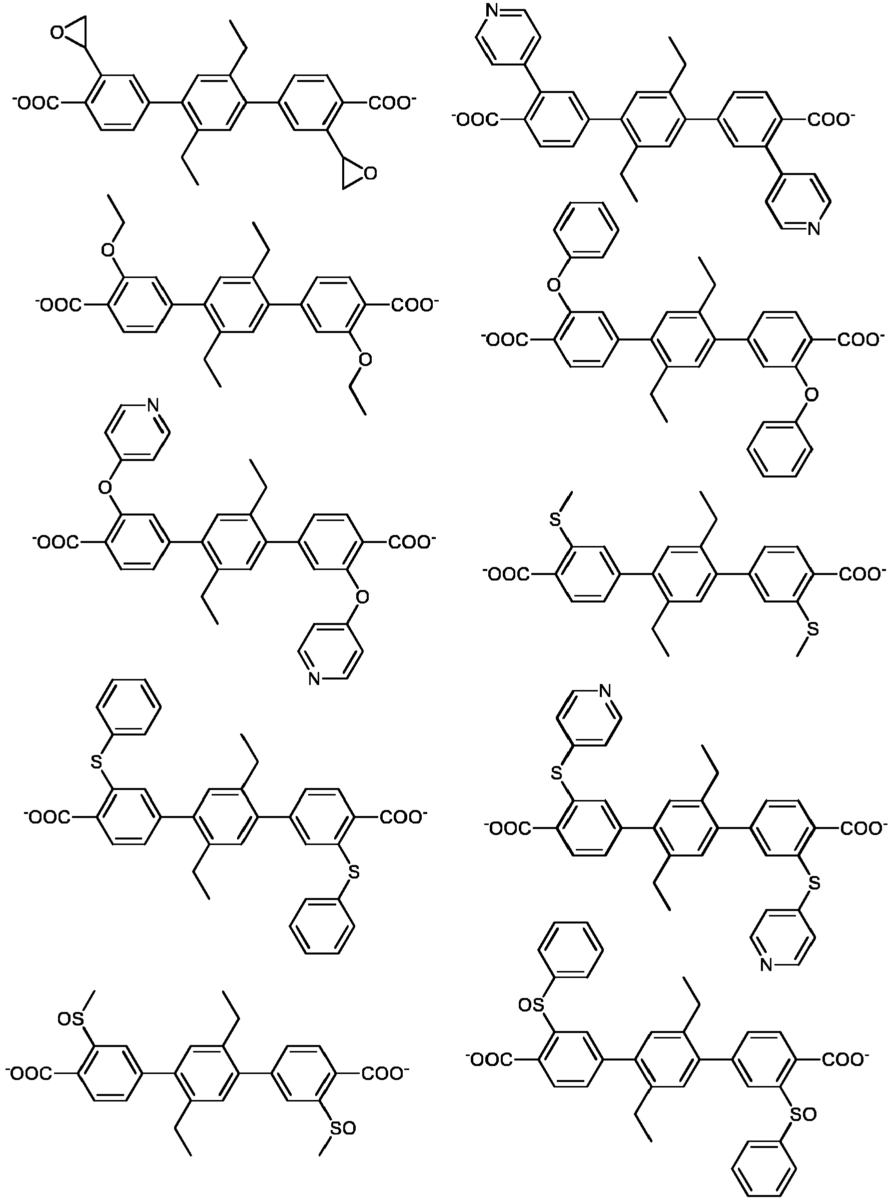

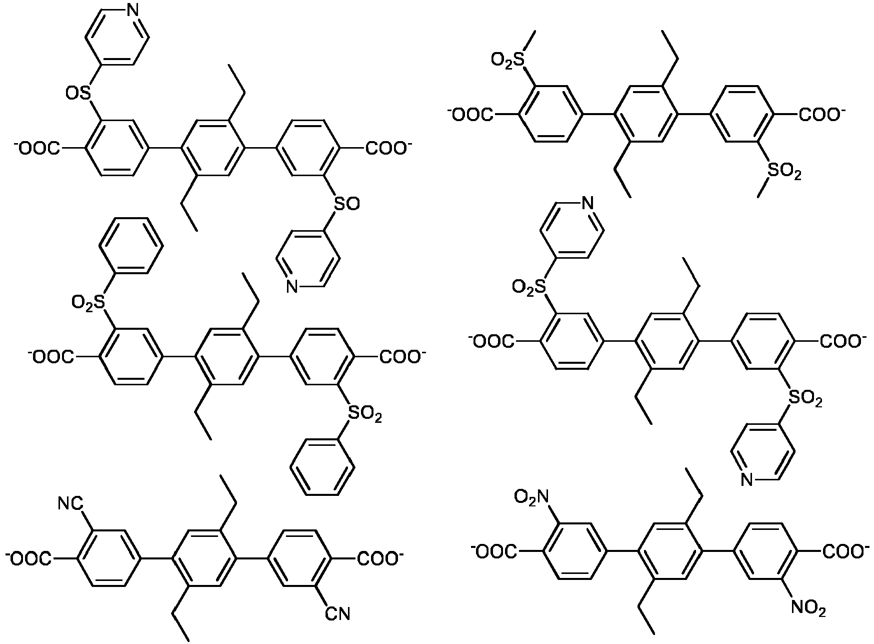

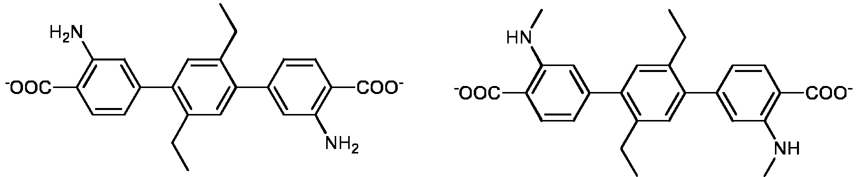

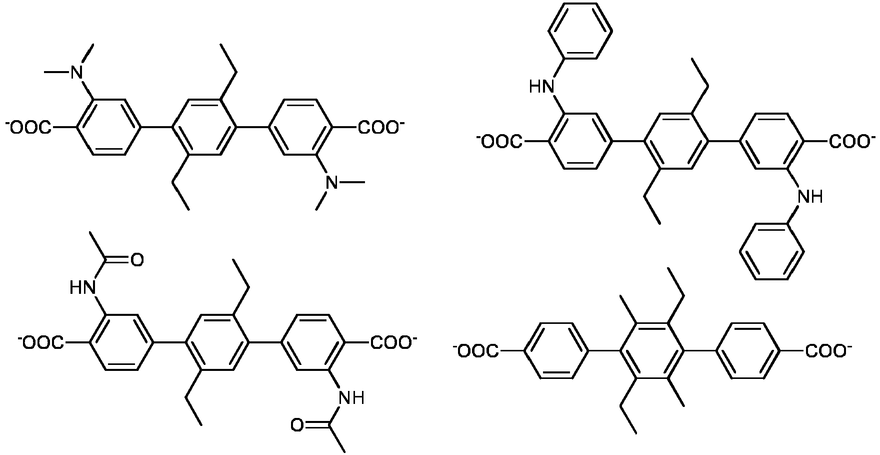

The metal-organic framework of the present invention may comprise an organic ligand other than the carboxylate ion of formula (I). As such an organic ligand, terephthalic acid, phthalic acid, isophthalic acid, 5-cyanoisophthalic acid, 1,3,5-trimesic acid, 1,3,5-tris(4-carboxyphenyl)benzene, 4,4'-dicarboxybiphenyl, 3,5-dicarboxypyridine, 2,3-dicarboxypyrazine, 1,3,5-tris(4-carboxyphenyl)benzene, 1,2,4,5-tetrakis(4-carboxyphenyl)benzene, 9,10-anthracendicarboxylic acid, 2,6-naphthalenedicarboxylic acid, [1,1': 4',1"] terphenyl-3,3" ,5,5"-tetracarboxylic acid, biphenyl-3,3",5,5"-tetracarboxylic acid, 3,3',5,5'-tetracarboxydiphenylmethane, 1,3,5-tris(4'-carboxy[1,1'-biphenyl]-4-yl)benzene, 1,3,5-tris(4-carboxyphenyl)triazine, 1,2-bis(4-carboxy-3-nitrophenyl)ethene, 1,2-bis(4-carboxy-3-aminophenyl)ethene, trans, trans-muconic acid, fumaric acid, benzimidazole, imidazole, 1,4-diazabicyclo[2.2.2]octane (DABCO), pyrazine, 4,4'-dipyridyl, 1,2-di(4-pyridyl)ethylene, 2,7-diazapyrene, 4,4'-azobispyridine, bis(3-(4-pyridyl)-2,4-pentanedionato)copper or the like is exemplified.

The mixing molar ratio when the carboxylate ion of formula (I) and the organic ligand other than the carboxylate ion of formula (I) are used is not particularly limited, but, for example, when the organic ligand other than the carboxylate ion of formula (I) is used as a pillar molecule, and bridging is performed with the pillar molecule to construct a three-dimensional structure like a pillared layer type, the organic ligand other than the carboxylate ion of formula (I) is preferably used in excess of the carboxylate ion of formula (I).

The method for producing the metal-organic framework of the present invention is not particularly limited, and it is possible to use any one of a solution method such as a solvent diffusion method, a solvent stirring method, or a hydrothermal method, a microwave method in which a reaction solution is irradiated with microwaves to uniformly heat the entire system in a short time, an ultrasonic method in which by irradiating a reaction container with ultrasonic waves, changes in pressure occur repeatedly in the reaction container, and due to these changes in pressure, a phenomenon called cavitation in which a solvent forms bubbles and collapses occurs, and at the time, energy fields as high as about 5000 K and 10000 bar are reaction fields for the production of crystals locally formed, a solid phase synthesis method in which a metal ion source and an organic ligand are mixed without using a solvent, a LAG (liquid assisted grinding) method in which a metal ion source and an organic ligand are mixed with water at the water-of-crystallization level added, and the like.

The production method includes, for example, the step of preparing each of a first solution containing a metal compound that is a source of a metal ion, and a solvent, a second solution containing a carboxylate ion of formula (I) or carboxylic acid, which is a precursor thereof, and a solvent, and a third solution containing a compound that is another polydentate ligand, and a solvent, as needed, and the step of mixing the first solution with the second solution and the third solution to prepare a reaction liquid, and heating this reaction liquid to obtain a metal-organic framework. The first to third solutions need not be separately prepared, and, for example, the above metal compound, the carboxylate ion of formula (I) or carboxylic acid, which is a precursor thereof, the compound that is another polydentate ligand, and the solvent may be mixed at one time to prepare one solution.

The mixing molar ratio between the above metal compound and the carboxylate ion of formula (I) or carboxylic acid, which is a precursor thereof, may be arbitrarily selected in accordance with the pore size and surface characteristics of the metal-organic framework to be obtained, but 1 mol or more of the metal compound is preferably used with respect to 1 mol of the carboxylate ion of formula (I) or carboxylic acid, which is a precursor thereof, and further 1.1 mol or more, further 1.2 mol or more, further 1.5 mol or more, further 2 mol or more, and further 3 mol or more of the metal compound is preferably used.

The concentration of the above metal ion in the reaction liquid is preferably in the range of 25 to 200 mol/L.

The concentration of the carboxylate ion of formula (I) or carboxylic acid, which is a precursor thereof, in the reaction liquid is preferably in the range of 10 to 100 mol/L.

The concentration of the organic ligand other than the carboxylate ion of formula (I) or carboxylic acid, which is a precursor thereof, in the reaction liquid is preferably 25 to 100 mol/L.

While not particularly limited, as the solvent used, one or more solvents selected from the group consisting of N,N-dimethylformamide (hereinafter sometimes described as "DMF"), N,N-diethylformamide (hereinafter sometimes described as "DEF"), N,N-dimethylacetamide (hereinafter sometimes described as "DMA"), and water may be used. It is preferable that among these, any of DMF, DEF, or DMA be used singly, or a DMF/water mixed solvent, a DEF/water mixed solvent, or a DMA/water mixed solvent be used.

The heating temperature of the reaction liquid is not particularly limited but is preferably in the range of room temperature to 140°C.

The metal-organic framework of the present invention may store a gas such as hydrogen, methane, acetylene, carbon dioxide, or nitrogen by adsorbing or occluding the gas.

The method for storing a gas using the metal-organic framework of the present invention is not particularly limited, but a method for bringing the metal-organic framework of the present invention and a gas into contact with each other is preferable, and the contact method is not particularly limited. Examples of the method include a method for filling a tank with the metal-organic framework of the present invention to provide a gas storage tank, and allowing a gas to flow into the tank, a method for supporting the metal-organic framework of the present invention on the surface constituting the inner wall of a tank to provide a gas storage tank, and allowing a gas to flow into the tank, and a method for molding a tank from a material comprising the metal-organic framework of the present invention to provide a gas storage tank, and allowing a gas to flow into the tank.

### Examples

The present invention will be described in detail below using Examples, but the present invention is in no way limited to the scope of the Examples.

As a carboxylic acid that served as a precursor of the carboxylate ion of formula (I) constituting the metal-organic framework of the present invention, organic ligands 1 to 19 shown in the following Table 1 were used.

**[Table 1]**

| | |
|---|---|
| Organic ligand 1 | Organic ligand 2 |
| 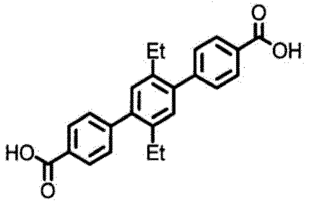 | 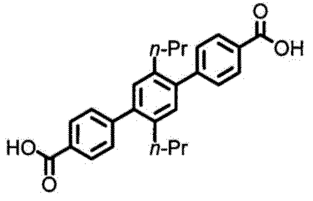 |
| Organic ligand 3 | Organic ligand 4 |
| 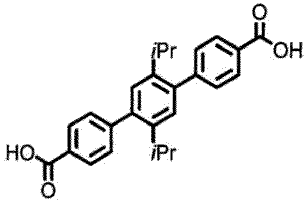 | 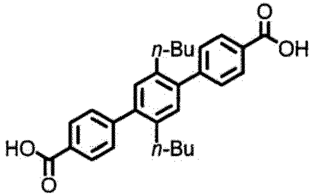 |
| Organic ligand 5 | Organic ligand 6 |
| 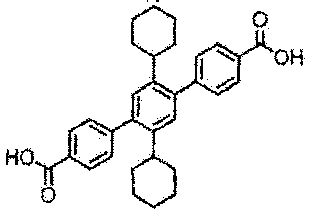 | 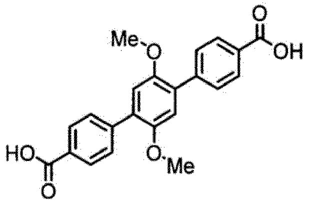 |
| Organic ligand 7 | Organic ligand 8 |
| 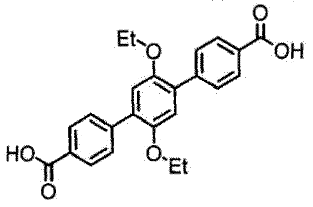 | 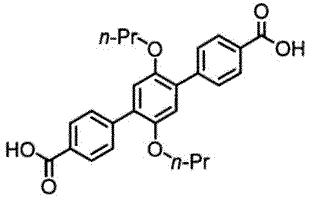 |
| Organic ligand 9 | Organic ligand 10 |
| 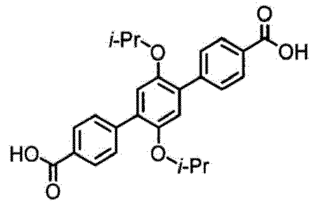 | 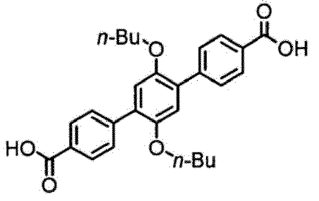 |
| Organic ligand 11 | Organic ligand 12 |
| 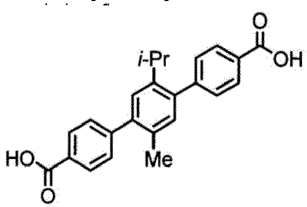 | 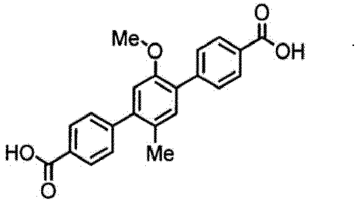 |
| Organic ligand 13 | Organic ligand 14 |
| 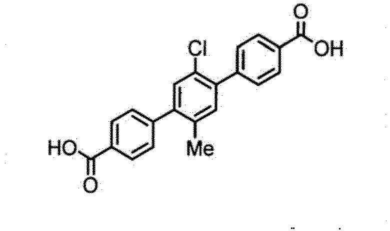 | 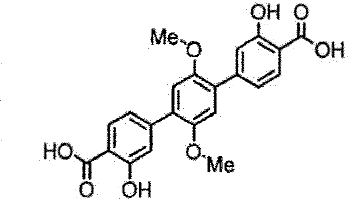 |
| Organic ligand 15 | Organic ligand 16 |
| 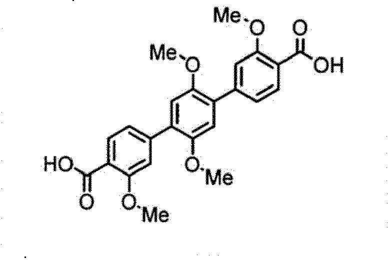 | 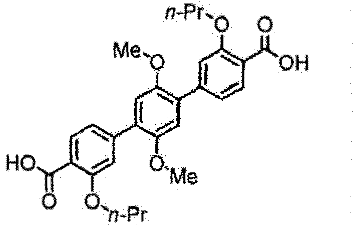 |
| Organic ligand 17 | Organic ligand 18 |
| 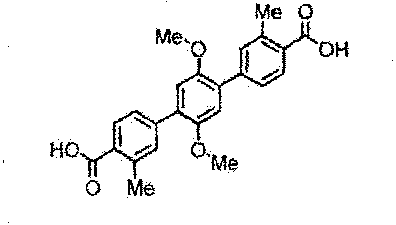 | 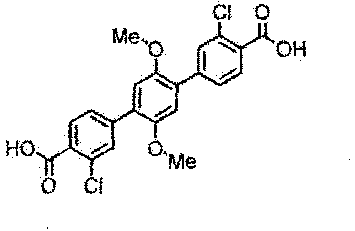 |
| Organic ligand 19 | |
| 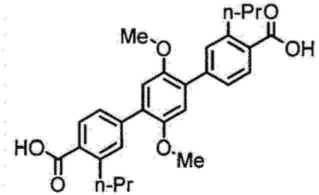 | |

### [Manufacturing Example 1] Synthesis of Organic Ligand 1

A solution of 1,4-dibromo-2,5-diethylbenzene (2.5 mmol), 4-methoxycarbonylphenylboronic acid (7.5 mmol), tetrakis(triphenylphosphine)palladium (0) (0.25 mmol), cesium fluoride (1.25 mmol), cesium carbonate (7.5 mmol), 25 mL of 1,4-dioxane, and 1 mL of water was heated at 100°C overnight under nitrogen. The reaction liquid was returned to room temperature, the liquid was separated by adding water, an organic layer extracted with dichloromethane was dried with magnesium sulfate and filtered, the solvent was distilled away at reduced pressure, and the obtained solid was purified using silica gel column chromatography (chloroform), thereby obtaining 2.1 mmol of 2',5'-diethyl[1,1':4',1"-terphenyl]-4,4"-dimethyl dicarboxylate as a colorless solid. A solution of 2.0 mmol of the obtained 2',5'-diethyl[1,1' :4',1"-terphenyl]-4,4"-dimethyl dicarboxylate, potassium hydroxide (20 mmol), 20 mL of tetrahydrofuran, and 10 mL of water was heated and refluxed for two days. The reaction liquid was returned to room temperature, tetrahydrofuran was distilled away, then, concentrated hydrochloric acid was added, a precipitated solid was filtered and washed with water, and the obtained solid was dried at 100°C, thereby obtaining 2.0 mmol of 2',5'-diethyl[1,1':4',1"-terphenyl]-4,4"-dicarboxylic acid as a colorless solid.

### [Manufacturing Example 2] to [Manufacturing Example 13]

The same operation as in Manufacturing Example 1 except that a reagent shown in the following Table 2 was used instead of 1,4-dibromo-2,5-diethylbenzene was performed, thereby obtaining organic ligands 2 to 13 all as colorless solids.

**[Table 2]**

| Manufacturing example | Reagent |
|---|---|
| 1 | 1,4-Dibromo-2,5-diethylbenzene |
| 2 | 1,4-Dibromo-2,5-di-n-propylbenzene |
| 3 | 1,4-Dibromo-2,5-diisopropylbenzene |
| 4 | 1,4-Dibromo-2,5-di-n-butylbenzene |
| 5 | 1,4-Dibromo-2,5-dicyclohexylbenzene |
| 6 | 1,4-Dibromo-2,5-dimethoxybenzene |
| 7 | 1,4-Dibromo-2,5-diethoxybenzene |
| 8 | 1,4-Dibromo-2,5-di-n-propoxybenzene |
| 9 | 1,4-Dibromo-2,5-diisopropoxybenzene |
| 10 | 1,4-Dibromo-2,5-di-n-butoxybenzene |
| 11 | 1,4-Dibromo-2-isopropyl-5-methylbenzene |
| 12 | 1,4-Dibromo-2-methoxy-5-methylbenzene |
| 13 | 1,4-Dibromo-2-chloro-5-methylbenzene |

### [Manufacturing Example 14]

The same operation as in Manufacturing Example 1 except that 1,4-dibromo-2,5-dimethoxylbenzene was used instead of 1,4-dibromo-2,5-diethylbenzene of Manufacturing Example 1 and 3-hydroxy-4-ethoxycarbonylphenylboronic acid was used instead of 4-methoxycarbonylphenylboronic acid was performed, and an organic ligand 14 was obtained as a colorless solid.

### [Manufacturing Example 15]

3,3"-Dihydroxy-2',5'-dimethoxy-[1,1':4',1"-terphenyl]-4,4"-diethyl dicarboxylate synthesized in Manufacturing Example 14 (1.0 mmol), potassium carbonate (3.0 mmol), and methyl iodide (4.0 mmol) were dissolved in 10 mL of DMF and stirred at 100°C overnight. The reaction liquid was returned to room temperature, water was added, an organic layer extracted with ethyl acetate was dried with magnesium sulfate and filtered, and the filtrate was distilled away at reduced pressure. The obtained solid was separated and purified by silica gel column chromatography (chloroform), thereby obtaining 2',3,3",5'-tetramethoxy-[1,1':4',1"-terphenyl]-4,4"-diethyl dicarboxylate as a colorless solid. Potassium hydroxide (10.0 mmol), 20 mL of ethanol, and 10 mL of water were added to the obtained colorless solid and stirred at 90°C overnight. The solution was returned to room temperature, a solid precipitated by adding dilute hydrochloric acid was filtered, well washed with water, and dried, thereby obtaining 2',3,3",5'-tetramethoxy-[1,1':4',1"-terphenyl]-4,4"-dicarboxylic acid of an organic ligand 15 as a colorless solid.

### [Manufacturing Example 16]

An organic ligand 16 was obtained as a colorless solid by the same operation as in Manufacturing Example 15 except that propyl iodide was used instead of methyl iodide used in Manufacturing Example 15.

### [Manufacturing Example 17] to [Manufacturing Example 19]

The same operation as in Manufacturing Example 14 except that a reagent shown in the following Table 3 was used was performed, thereby obtaining organic ligands 17 to 19 all as colorless solids.

**[Table 3]**

| Manufacturing example | Reagent |
|---|---|
| 17 | 3-Methyl-4-ethoxycarbonylphenylboronic acid |
| 18 | 3-Chloro-4-ethoxycarbonylphenylboronic acid |
| 19 | 3-n-Propyl-4-ethoxycarbonylphenylboronic acid |

### [Example 1-1]

A solution obtained by adding 6 mL of DMF to the organic ligand 1 (0.3 mmol) and zinc nitrate hexahydrate (0.6 mmol) was heated in an oven (reaction conditions: 120°C, 24 hours). The reaction liquid was returned to room temperature, and the supernatant was removed. The residue was washed using 10 mL of DMF, the solvent was removed, 10 mL of chloroform was added to the residue and immersed overnight. A solid obtained by removing chloroform from the residue-immersed liquid was dried at 150°C in a vacuum for 5 hours, thereby obtaining a metal-organic framework 1-1 as a colorless solid.

### [Example 1-2] to [Example 1-16]

Metal-organic frameworks 1-2 to 1-16 were obtained by performing operations similar to those of Example 1-1 except that the organic ligands and the solvents shown in the following Table 4 were used, and the reaction was performed under the reaction conditions shown in Table 4. The results are shown in Table 4.

**[Table 4]**

| Example | Organic ligand | Solvent | Temperature (°C) | Heating time (hr) | Property |
|---|---|---|---|---|---|
| 1-1 | 1 | DMF | 120 | 24 | Colorless solid |
| 1-2 | 1 | DEF | 120 | 24 | Slightly yellow solid |
| 1-3 | 1 | DMF | 90 | 12 | Colorless solid |
| 1-4 | 2 | DMF | 90 | 24 | Colorless solid |
| 1-5 | 2 | DMF | 120 | 24 | Colorless solid |
| 1-6 | 2 | DEF | 90 | 24 | Colorless solid |
| 1-7 | 2 | DEF | 120 | 24 | Colorless solid |
| 1-8 | 3 | DMF | 90 | 24 | Colorless solid |
| 1-9 | 3 | DMF | 120 | 24 | Colorless solid |
| 1-10 | 3 | DEF | 90 | 24 | Colorless solid |
| 1-11 | 3 | DEF | 120 | 24 | Colorless solid |
| 1-12 | 14 | DMF | 120 | 24 | Brownish yellow solid |
| 1-13 | 15 | DMF | 120 | 24 | Off-white powder |
| 1-14 | 16 | DMF | 120 | 24 | Off-white powder |
| 1-15 | 17 | DMF | 120 | 24 | Off-white powder |
| 1-16 | 19 | DMF | 120 | 24 | Off-white powder |

### [Example 2-1]

A solution obtained by adding 7.5 mL of DMF as a solvent to the organic ligand 1 (0.375 mmol), zinc nitrate hexahydrate (0.375 mmol), and 1,4-diazabicyclo[2.2.2]octane (0.20 mmol) as an auxiliary ligand was stirred for 15 minutes and then irradiated with ultrasonic waves. A solution obtained by filtering the liquid mixture was heated in an oven (reaction conditions: 120°C, 48 hours). The reaction liquid was returned to room temperature, and the precipitated solid was washed with 10 mL of DMF three times and further washed with 10 mL of chloroform three times. The obtained solid was immersed in 10 mL of chloroform overnight, and the solid obtained by pressure filtration was dried at 150°C in a vacuum for 5 hours, thereby obtaining a metal-organic framework 2-1 as a colorless solid.

### [Example 2-2]

The same operation as in Example 2-1 was performed except that the organic ligand 2 was used instead of the organic ligand 1, thereby obtaining a metal-organic framework 2-2 as a milky green solid.

### [Example 3-1]

The organic ligand 1 (0.5 mmol) was dissolved in 7 mL of DMF, triethylamine (2.0 mmol) was added thereto, furthermore, a DMF (8 mL) solution of zinc acetate dihydrate (1.27 mmol) was added dropwise, and the obtained solution was stirred at room temperature for 20 hours and left to stand. The reaction liquid was centrifugally separated, then, the supernatant was removed, and the obtained solid was immersed in 20 mL of DMF overnight. After that, an operation in which the supernatant of the solid-immersed liquid was removed, the obtained solid was immersed in 20 mL of chloroform overnight, and then the solid was separated was repeated three times. After that, the separated solid was dried at 150°C in a vacuum for 5 hours, thereby obtaining a metal-organic framework 3-1 as a light gray powder.

### [Example 3-2] to [Example 3-11]

The same operation as in Example 3-1 was performed except that a reaction was performed using an organic ligand shown in the following Table 5 under a reaction condition shown in Table 5, thereby obtaining metal-organic frameworks 3-2 to 3-11. The results are shown in Table 5.

**[Table 5]**

| Example | Organic ligand | Base | Stirring time (hr) | Property |
|---|---|---|---|---|
| 3-1 | 1 | Et₃N | 20 | Light gray powder |
| 3-2 | 1 | None | 2.5 | Colorless powder |
| 3-3 | 2 | None | 2.5 | Colorless powder |
| 3-4 | 2 | Et₃N | 2.5 | Colorless powder |
| 3-5 | 3 | None | 2.5 | Colorless powder |
| 3-6 | 5 | None | 2.5 | Colorless powder |
| 3-7 | 6 | None | 2.5 | Colorless powder |
| 3-8 | 7 | None | 2.5 | Colorless powder |
| 3-9 | 8 | None | 2.5 | Colorless powder |
| 3-10 | 9 | None | 2.5 | Colorless powder |
| 3-11 | 10 | None | 2.5 | Colorless powder |

### [Example 4-1]

0.5 mL of triethylamine was added dropwise under stirring to a DMF (10 mL) solution of the organic ligand 1 (0.3 mmol) and zinc nitrate hexahydrate (0.4 mmol) and stirred at room temperature for five minutes. The reaction mixture was centrifugally separated, then, the supernatant was removed, 10 mL of DMF was added to the residue, centrifugal separation was performed again for 10 minutes, and the supernatant was removed. The residue was immersed in DMF overnight, then, centrifugal separation was performed for 10 minutes, the supernatant was removed, and 10 mL of chloroform was added to the residue. An operation in which a solid obtained by further centrifugally separating the residue-immersed liquid was immersed in 20 mL of chloroform overnight and then centrifugally separated was repeated three times. After that, a solid obtained by centrifugal separation was dried at 150°C in a vacuum for 5 hours, thereby obtaining a metal-organic framework 4-1 as a thin gray powder.

### [Example 4-2]

A metal-organic framework 4-2 was obtained as a colorless powder by the same operation as in Example 4-1 except that the organic ligand 2 was used instead of the organic ligand 1.

### [Example 4-3] to [Example 4-8]

The same operation as in Example 4-1 was performed except that a reaction was performed using an organic ligand shown in the following Table 6 under a reaction condition shown in Table 6, thereby obtaining metal-organic frameworks 4-3 to 4-8. The results are shown in Table 6.

**[Table 6]**

| Example | Organic ligand | Property |
|---|---|---|
| 4-3 | 5 | Colorless powder |
| 4-4 | 6 | Colorless powder |
| 4-5 | 7 | Colorless powder |
| 4-6 | 8 | Colorless powder |
| 4-7 | 9 | Colorless powder |
| 4-8 | 10 | Colorless powder |

### [Example 5-1]

A solution obtained by adding 4 mL of DMF to the organic ligand 1 (0.2 mmol) and copper nitrate trihydrate (0.2 mmol) was irradiated with ultrasonic waves for 15 minutes. A liquid obtained by filtering the solution was heated at 120°C for 25 hours. The reaction liquid was returned to room temperature, then, centrifugal separation was performed for 10 minutes, the supernatant was removed, 10 mL of DMF was added to the residue, centrifugal separation was performed again for 10 minutes, and the supernatant was removed. The residue was immersed in DMF overnight, then, centrifugal separation was performed for 10 minutes, DMF was removed by pressure filtration, and then 10 mL of chloroform was added to the obtained solid. An operation in which a solid obtained by further centrifugally separating the solid-immersed liquid was immersed in 20 mL of chloroform overnight and then centrifugally separated was repeated three times. After that, a solid obtained by pressure filtration was dried at 150°C in a vacuum for 5 hours, thereby obtaining a metal-organic framework 5-1.

### [Example 5-2] to [Example 5-7]

The same operation as in Example 5-1 was performed except that a reaction was performed using an organic ligand shown in the following Table 7 under a reaction condition shown in Table 7, thereby obtaining metal-organic frameworks 5-2 to 5-7. The results are shown in Table 7.

**[Table 7]**

| Example | Organic ligand | Solvent | Temperature (°C) | Heating time (hr) | Property |
|---|---|---|---|---|---|
| 5-1 | 1 | DMF | 120 | 25 | Green solid |
| 5-2 | 1 | DMF | 90 | 25 | Watery solid |
| 5-3 | 1 | DEF | 120 | 25 | Watery solid |
| 5-4 | 1 | DEF | 90 | 25 | Watery solid |
| 5-5 | 2 | DMF | 120 | 40 | Purple solid |
| 5-6 | 2 | DMF | 90 | 40 | Purple solid |
| 5-7 | 4 | DMF | 120 | 24 | Purple solid |

### [Example 6-1]

A solution of 9 mL of THF of the organic ligand 1 (0.4 mmol) and nickel nitrate hexahydrate (0.8 mmol) and 1 mL of water were heated at 100°C for 48 hours. The reaction liquid was returned to room temperature, the supernatant was removed, DMF was added to the residue, and the supernatant was removed again. After that, chloroform was added to the residue, the residue was immersed overnight, a solid obtained by the pressure filtration of the residue-immersed liquid was dried at 150°C in a vacuum for 5 hours, thereby obtaining a metal-organic framework 6-1 as a brownish yellow powder.

### [Example 6-2]

A metal-organic framework 6-2 (slightly green powder) was obtained by the same operation as in Example 6-1 except that the organic ligand 2 was used instead of the organic ligand 1.

### [Example 6-3]

A metal-organic framework 6-3 (slightly green powder) was obtained by the same operation as in Example 6-1 except that the organic ligand 4 was used instead of the organic ligand 1.

### [Example 7-1]

A solution of the organic ligand 1 (0.3 mmol), zirconium chloride (0.3 mmol), water (3.6 mmol), acetic acid (9 mmol), and 4 mL of DMF was irradiated with ultrasonic waves. After that, the solution was heated at 120°C for 24 hours. The reaction liquid was returned to room temperature, then, centrifugal separation was performed for 10 minutes, and the supernatant was removed. DMF was added to the residue, centrifugal separation was performed again for 10 minutes, and the supernatant was removed. The residue was immersed in DMF overnight and then centrifugally separated for 10 minutes, and DMF was removed. An operation in which 10 mL of acetone was added to the residue, the residue was centrifugally separated, and the supernatant was removed was repeated three times, acetone was added again, the residue was immersed overnight and then centrifugally separated, and the obtained solid was dried at 150°C in a vacuum for 5 hours, thereby obtaining a metal-organic framework 7-1 as a colorless solid.

### [Example 7-2] and [Example 7-3]

The same operation as in Example 7-1 was performed except that a reaction was performed with an organic ligand shown in the following Table 8, thereby obtaining metal-organic frameworks 7-2 and 7-3. The results are shown in Table 8.

**[Table 8]**

| Example | Organic ligand | Property |
|---|---|---|
| 7-1 | 1 | Colorless powder |
| 7-2 | 2 | Colorless powder |
| 7-3 | 4 | Gray powder |

### [Example 7-4]

Zirconium chloride, water, and acetic acid were dissolved in DMF, ultrasonic waves were applied to the solution, and the solution was left to stand at room temperature for 2 days. After that, the organic ligand 3 was added thereto, and then the same treatment as in Example 7-1 was performed, thereby obtaining a metal-organic framework 7-4 (colorless powder).

### [Example 7-5]

The same operation as in Example 7-4 was performed except that water was not added, thereby obtaining a metal-organic framework 7-5 (colorless powder).

### [Example 7-6] to [Example 7-25]

The same operation as in Example 7-1 was performed except that a reaction was performed with an organic ligand shown in the following Table 9, thereby obtaining metal-organic frameworks 7-6 to 7-25. The results are shown in Table 9.

**[Table 9]**

| Example | Organic ligand | Stirring while heating | Acid | Property |
|---|---|---|---|---|
| 7-6 | 2 | No | Benzoic acid | Colorless crystal |
| 7-7 | 3 | No | Acetic acid | Colorless powder |
| 7-8 | 3 | Yes | Acetic acid | Colorless powder |
| 7-9 | 5 | No | Acetic acid | Colorless powder |
| 7-10 | 6 | No | Acetic acid | Colorless powder |
| 7-11 | 7 | No | Acetic acid | Colorless powder |
| 7-12 | 8 | No | Acetic acid | Colorless powder |
| 7-13 | 8 | Yes | Acetic acid | Colorless powder |
| 7-14 | 9 | No | Acetic acid | Colorless powder |
| 7-15 | 10 | No | Acetic acid | Colorless powder |
| 7-16 | 11 | No | Acetic acid | Colorless powder |
| 7-17 | 12 | No | Acetic acid | Gray powder |
| 7-18 | 13 | No | Acetic acid | Colorless powder |
| 7-19 | 15 | No | Acetic acid | Colorless solid |
| 7-20 | 16 | No | Acetic acid | Off-white powder |
| 7-21 | 17 | No | Acetic acid | Colorless solid |
| 7-22 | 19 | No | Acetic acid | Colorless solid |
| 7-23 | 14 | No | Acetic acid | Colorless solid |
| 7-24 | 18 | No | Acetic acid | Colorless solid |
| 7-25 | 16 | No | Acetic acid | Colorless solid |

### [Example 8-1]

A solution of the organic ligand 1 (0.175 mmol), zirconium chloride oxide octahydrate (0.175 mmol), and 4 mL of a solvent mixture of formic acid and DMF (1:7) was irradiated with ultrasonic waves. After that, the solution was heated at 120°C for 24 hours. The reaction liquid was returned to room temperature, and then the supernatant was removed. An operation in which DMF was added to the residue, centrifugal separation was performed again for 10 minutes, and the supernatant was removed was repeated three times, then, DMF was added, and the residue was immersed overnight. An operation in which DMF was removed from the residue-immersed liquid, then, methanol was added to the residue, the residue was centrifugally separated, and the supernatant was removed was repeated three times. Methanol was added again to the residue, the residue was immersed overnight, then, methanol was removed, and the obtained solid was dried at 150°C in a vacuum for 5 hours, thereby obtaining a metal-organic framework 8-1 as a light gray powder.

### [Example 9-1]

The organic ligand 2 (0.36 mmol), tetraisopropyl orthotitanate (0.074 mmol), 2.2 g of acetic acid, and 7 mL of DMF were mixed, and ultrasonic waves were applied thereto. After that, the mixture was heated at 120°C for 48 hours. The mixture was returned to room temperature, then, centrifugal separation was performed for 10 minutes, and the supernatant was removed. DMF was added to the residue, centrifugal separation was performed again for 10 minutes, and the supernatant was removed. The residue was immersed in DMF overnight, then, centrifugal separation was performed for 10 minutes, DMF was removed, 10 mL of acetone was added, and centrifugal separation and supernatant removal were repeated three times. Acetone was added again, the residue was immersed overnight, and the centrifugally separated solid was dried at 150°C in a vacuum for 5 hours, thereby obtaining a metal-organic framework 9-1 as an off-white powder.

### [Example 9-2] to [Example 9-6]

The same operation as in Example 9-1 was performed except that a reaction was performed with an organic ligand shown in the following Table 10, thereby obtaining metal-organic frameworks 9-2 to 9-6. The results are shown in Table 10.

**[Table 10]**

| Example | Organic ligand | Property |
|---|---|---|
| 9-2 | 3 | Off-white powder |
| 9-3 | 5 | Colorless powder |
| 9-4 | 6 | Slightly yellow powder |
| 9-5 | 10 | Slightly yellow powder |
| 9-6 | 12 | Gray powder |

### [Example 10-1]

5 mL of DMF was added to the organic ligand 2 (0.5 mmol) and chromium chloride hexahydrate (0.5 mmol), and ultrasonic waves were applied thereto. The solution was heated at 190°C for 24 hours. The mixture was returned to room temperature, then, centrifugal separation was performed for 10 minutes, and the supernatant was removed. 10 mL of DMF was added to the residue, centrifugal separation was performed again for 10 minutes, and the supernatant was removed. The residue was immersed in DMF overnight, then, centrifugal separation was performed for 10 minutes, DMF was removed by pressure filtration, and then 10 mL of chloroform was added. A washing operation in which the centrifugally separated solid was immersed in 20 mL of chloroform overnight and centrifugally separated again was repeated three times. After that, a solid obtained by pressure filtration was dried at 150°C in a vacuum for 5 hours, thereby obtaining a metal-organic framework 10-1 as a green solid.

### [Example 10-2] to [Example 10-4]

The same operation as in Example 10-1 was performed except that a reaction was performed with an organic ligand shown in the following Table 11, thereby obtaining metal-organic frameworks 10-2 to 10-4. The results are shown in Table 11.

**[Table 11]**

| Example | Organic ligand | Heating time (hr) | Property |
|---|---|---|---|
| 10-2 | 3 | 72 | Green solid |
| 10-3 | 6 | 24 | Green solid |
| 10-4 | 8 | 24 | Green solid |

### [Example 11-1]

5 mL of DMF was added to the organic ligand 2 (0.18 mmol) and aluminum nitrate nonahydrate (0.24 mmol), and ultrasonic waves were applied thereto. The solution was heated at 120°C for 24 hours. The mixture was returned to room temperature, then, centrifugal separation was performed for 10 minutes, and the supernatant was removed. 10 mL of DMF was added to the residue, centrifugal separation was performed again for 10 minutes, and the supernatant was removed. The residue was immersed in DMF overnight, then, centrifugal separation was performed for 10 minutes, DMF was removed by pressure filtration, and then 10 mL of acetone was added. A washing operation in which the centrifugally separated solid was immersed in 20 mL of acetone overnight and centrifugally separated again was repeated three times. After that, a solid obtained by pressure filtration was dried at 150°C in a vacuum for 5 hours, thereby obtaining a metal-organic framework 11-1 as a colorless powder.

### [Example 11-2] to [Example 11-14]

The same operation as in Example 11-1 was performed except that a reaction was performed with an organic ligand shown in the following Table 12, thereby obtaining metal-organic frameworks 11-2 to 11-14. The results are shown in Table 12.

**[Table 12]**

| Example | Organic ligand | Heating time (hr) | Stirring | Solvent | Temperature (°C) | Property |
|---|---|---|---|---|---|---|
| 11-2 | 3 | 24 | No | DMF | 120 | Colorless powder |
| 11-3 | 5 | 24 | No | DMF | 120 | Colorless powder |
| 11-4 | 6 | 24 | No | DMF | 120 | Colorless powder |
| 11-5 | 8 | 24 | No | DMF | 120 | Colorless powder |
| 11-6 | 8 | 42 | No | H₂O | 220 | Off-white powder |
| 11-7 | 10 | 24 | No | DMF | 120 | Colorless powder |
| 11-8 | 10 | 24 | Yes | DMF | 120 | Colorless powder |
| 11-9 | 15 | 72 | No | DMF | 120 | Slightly brown solid |
| 11-10 | 16 | 72 | No | DMF | 120 | Colorless solid |
| 11-11 | 17 | 72 | No | DMF | 120 | Colorless solid |
| 11-12 | 19 | 72 | No | DMF | 120 | Colorless powder |
| 11-13 | 18 | 72 | No | DMF | 120 | Colorless powder |
| 11-14 | 14 | 72 | No | DMF | 120 | Brown powder |

### [Example 12-1]

8 mL of DMF and 1 mL of acetic acid were added to the organic ligand 8 (0.1 mmol) and iron (III) chloride (0.25 mmol), and ultrasonic waves were applied thereto. The solution was heated at 150°C for 168 hours. The mixture was returned to room temperature, then, centrifugal separation was performed for 10 minutes, and the supernatant was removed. 10 mL of DMF was added to the residue, centrifugal separation was performed again for 10 minutes, and the supernatant was removed. The residue was immersed in DMF overnight, then, centrifugal separation was performed for 10 minutes, DMF was removed by pressure filtration, and then 10 mL of chloroform was added. A washing operation in which the centrifugally separated solid was immersed in 20 mL of chloroform overnight and centrifugally separated again was repeated three times. After that, a solid obtained by pressure filtration was dried at 150°C in a vacuum for 5 hours, thereby obtaining a metal-organic framework 12-1 as a blown powder.

### [Example 12-2] to [Example 12-4]

The same operation as in Example 12-1 was performed except that a reaction was performed with an organic ligand shown in the following Table 13, thereby obtaining metal-organic frameworks 12-2 to 12-4. The results are shown in Table 13.

**[Table 13]**

| Example | Organic ligand | Heating time (hr) | Metal (equivalent) | Acid | Property |
|---|---|---|---|---|---|
| 12-2 | 8 | 168 | Iron nitrate nonahydrate (1) | Acetic acid | Brown powder |
| 12-3 | 10 | 18 | Iron chloride hexahydrate (1) | TFA | Orange powder |
| 12-4 | 10 | 18 | Iron chloride hexahydrate (1/3) | TFA | Orange powder |

### [BET Specific Surface Area Measurement and Measurement of Amount of Hydrogen Stored]

For some of the obtained metal-organic frameworks, the BET specific surface area and the amount of hydrogen stored at 77 K-atmospheric pressure were measured.

The measurement of the BET specific surface area and the amount of hydrogen stored at 77 K-atmospheric pressure was performed using an apparatus for measuring the amount of gas adsorbed, Tristar-II (manufactured by Micromeritics Instrument Corporation).

The BET specific surface area was calculated by the following method. Approximately 50 mg of a metal-organic framework was placed inside a glass cell. The pressure inside the glass cell was reduced to a vacuum at a temperature of 135°C, and the inside of the glass cell was dried for 6 hours. The glass cell was mounted in the apparatus for measuring the amount of gas adsorbed, and immersed in a constant temperature bath containing liquid nitrogen. The pressure of nitrogen contained in the glass cell was gradually increased. The measurement was performed until the pressure of nitrogen introduced inside the glass cell reached 1.0 × 10⁵ Pa.

The amount of hydrogen stored at a temperature of 77 K and normal pressure was calculated by the following method. After the measurement of nitrogen, the gas species was changed to hydrogen, and the measurement was performed. The pressure of hydrogen contained in the glass cell was gradually increased. The measurement was performed until the pressure of hydrogen introduced inside the glass cell reached 1.0 × 10⁵ Pa.

The results of the measured BET specific surface area and the amount of hydrogen stored measured at 77 K-atmospheric pressure were shown in Table 14. "-" in the table means that no measurement was performed.

**[Table 14]**

| Organic metal framework No. | BET specific surface area (m²/g) | Amount of hydrogen stored (wt %) |
|---|---|---|
| 1-1 | 545 | - |
| 1-2 | 19.4 | - |
| 1-3 | 3.94 | - |
| 1-4 | 62.7 | - |
| 1-5 | 25 | - |
| 1-6 | 15.2 | - |
| 1-7 | 34 | - |
| 1-8 | 188 | - |
| 1-9 | 244 | - |
| 1-10 | 59 | - |
| 1-11 | 38.3 | - |
| 1-12 | 971 | 0.802 |
| 1-13 | 1 | 0.077 |
| 1-14 | 3 | 0.080 |
| 1-15 | 11 | 0.721 |
| 1-16 | 5 | 0.113 |
| 2-1 | 174 | 0.400 |
| 2-2 | 451 | 0.606 |
| 3-1 | 1256 | 0.933 |
| 3-2 | 1471 | 1.110 |
| 3-3 | 103 | 0.357 |
| 3-4 | 403 | 0.564 |
| 3-5 | 463 | 1.037 |
| 3-6 | 788 | 0.878 |
| 3-7 | 210 | 0.702 |
| 3-8 | 532 | 0.651 |
| 3-9 | 21 | 0.412 |
| 3-10 | 788 | 0.791 |
| 3-11 | 7 | 0.204 |
| 4-1 | 233 | 0.444 |
| 4-2 | 314 | 0.438 |
| 4-3 | 717 | 0.835 |
| 4-4 | 58 | 0.446 |
| 4-5 | 180 | 0.403 |
| 4-6 | 40 | 0.356 |
| 4-7 | 128 | 0.568 |
| 4-8 | 59 | 0.399 |
| 5-1 | 13.1 | 0.180 |
| 5-2 | 13.2 | 0.369 |
| 5-3 | 16.3 | 0.348 |
| 5-4 | 12.9 | 0.379 |
| 5-5 | 470 | 0.729 |
| 5-6 | 599 | 0.797 |
| 5-7 | - | 0.451 |
| 6-1 | 7.39 | 0.434 |
| 6-2 | 587 | 0.850 |
| 6-3 | 336 | 0.863 |
| 7-1 | 3014 | 1.482 |
| 7-2 | 2652 | 1.476 |
| 7-3 | 2592 | 1.549 |
| 7-4 | 2840 | 1.740 |
| 7-5 | 3046 | 1.707 |
| 7-6 | 2793 | 1.561 |
| 7-7 | 3038 | 1.860 |
| 7-8 | 2871 | 1.873 |
| 7-9 | 2146 | 1.830 |
| 7-10 | 3090 | 1.786 |
| 7-11 | 460 | 1.048 |
| 7-12 | 2628 | 1.889 |
| 7-13 | 2367 | 1.420 |
| 7-14 | 2480 | 1.540 |
| 7-15 | 2335 | 1.526 |
| 7-16 | 2603 | 1.420 |
| 7-17 | 3331 | 1.453 |
| 7-18 | 2854 | 1.159 |
| 7-19 | 939 | 0.837 |
| 7-20 | - | - |
| 7-21 | 1523 | 1.072 |
| 7-22 | 1070 | 1.168 |
| 7-23 | 621 | 0.688 |
| 7-24 | 198 | 0.438 |
| 7-25 | 425 | 1.050 |
| 8-1 | 3.09 | 0.235 |
| 9-1 | 818 | 0.632 |
| 9-2 | 858 | 0.710 |
| 9-3 | 593 | 0.709 |
| 9-4 | 1030 | 0.726 |
| 9-5 | 787 | 0.549 |
| 9-6 | 1038 | 0.686 |
| 10-1 | 563 | 0.651 |
| 10-2 | 582 | 0.802 |
| 10-3 | 428 | 0.643 |
| 10-4 | 2 | 0.031 |
| 11-1 | 1140 | 0.984 |
| 11-2 | 1557 | 1.367 |
| 11-3 | 1343 | 1.405 |
| 11-4 | 924 | 1.054 |
| 11-5 | 1018 | 1.041 |
| 11-6 | 129 | 0.464 |
| 11-7 | 1545 | 1.227 |
| 11-8 | 1602 | 1.145 |
| 11-9 | 946 | 0.880 |
| 11-10 | 677 | 0.576 |
| 11-11 | 1168 | 1.190 |
| 11-12 | 791 | 0.693 |
| 11-13 | 708 | 0.831 |
| 11-14 | 447 | 0.659 |
| 12-1 | - | 0.243 |
| 12-2 | - | 0.534 |
| 12-3 | 13 | 0.172 |
| 12-4 | 28 | 0.202 |

### [Single Crystal X-Ray Structure Analysis]

For each of the metal-organic frameworks 1-2 and 2-2 obtained in Examples 1-2 and 2-2, X-ray structure analysis was performed under the measurement conditions shown below.

### [Measurement Conditions]

One 0.1 × 0.05 × 0.05 mm colorless and transparent single crystal of the metal-organic framework 1-2 obtained in Example 1-2 was put on a micromount, and diffraction data was acquired by irradiating the single crystal with X-rays having a wavelength of 0.78192 Å using a single crystal X-ray analysis apparatus (D8 VENTURE, manufactured by Bruker) .

The obtained diffraction data was analyzed to determine the structure. The results are shown in Table 15.

**[Table 15]**

| | |
|---|---|
| Measurement temperature/°C | -173°C |
| Crystal size/mm | 0.10x0.05x0.05 |
| Color of crystal | Colorless |
| Chemical formula | C₁₅H_{8.5}N_{0.5}O₃Zn_{0.75} |
| Molecular weight | 292.75 |
| Crystal system | monoclinic |
| Space group | *l2lm* |
| a/Å | 6.0704(4) |
| b/Å | 27.533(2) |
| c/Å | 26.3841(19) |
| α/° | 90 |
| β/° | 88.881(6) |
| γ/° | 90 |
| V/Å³ | 4408.9(5) |
| Z | 8 |
| Reflections collected | 19832 |
| Independent reflections | 5078 |

For the metal-organic framework 2-2 obtained in Example 2-2, X-ray structure analysis was performed under conditions similar to those of the measurement shown above. The results are shown in Table 16.

**[Table 16]**

| | |
|---|---|
| Measurement temperature/°C | -173°C |
| Crystal size/mm | 0.02x0.01x0.01 |
| Color of crystal | Colorless |
| Chemical formula | C₄₀H₂₆N₃O₅Zn |
| Molecular weight | 694.01 |
| Crystal system | Monoclinic |
| Space group | C2/c |
| a/Å | 25.493(15) |
| b/Å | 28.775(17) |
| c/Å | 9.384(6) |
| β/° | 96.999(7) |
| V/Å³ | 6832(7) |
| Z | 8 |
| Reflections collected | 76628 |
| Independent reflections | 7836 |

### Industrial Applicability

The metal-organic framework of the present invention may store a gas such as hydrogen at a practical level. As a result, for example, the utilization of hydrogen is easier for the arrival of a hydrogen society.

## Claims

1. A metal-organic framework comprising a carboxylate ion of formula (I) and a multivalent metal ion bonded to each other, 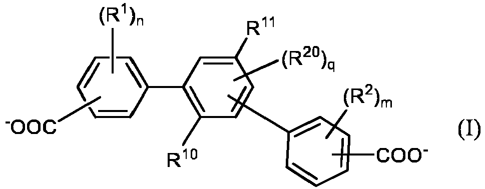 (in formula (I),
R¹ and R² each independently are a hydroxy group, an unsubstituted or substituted C1-6 alkyl group, an unsubstituted or substituted C3-8 cycloalkyl group, an unsubstituted or substituted C6-10 aryl group, an unsubstituted or substituted three- to six-membered heterocyclyl group, an unsubstituted or substituted C1-6 alkoxy group, an unsubstituted or substituted C6-10 aryloxy group, an unsubstituted or substituted heteroaryloxy group, a halogeno group, an unsubstituted or substituted C1-6 alkylthio group, an unsubstituted or substituted C6-10 arylthio group, an unsubstituted or substituted heteroarylthio group, an unsubstituted or substituted C1-6 alkylsulfinyl group, an unsubstituted or substituted C6-10 arylsulfinyl group, an unsubstituted or substituted heteroarylsulfinyl group, an unsubstituted or substituted C1-6 alkylsulfonyl group, an unsubstituted or substituted C6-10 arylsulfonyl group, an unsubstituted or substituted heteroarylsulfonyl group, a cyano group, a nitro group, or a group of formula (II): 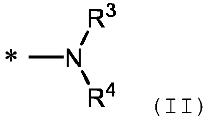
(in formula (II), R³ and R⁴ each independently are a hydrogen atom, an unsubstituted or substituted C1-6 alkyl group, an unsubstituted or substituted C6-10 aryl group, an unsubstituted or substituted C1-6 alkylcarbonyl group, or an unsubstituted or substituted C6-10 arylcarbonyl group, and * is a bonding site),
n is the number of R¹ and is 0, 1, 2, 3, or 4, and when n is 2 or more, a plurality of R¹s are the same or different from each other,
m is the number of R² and is 0, 1, 2, 3, or 4, and when m is 2 or more, a plurality of R²s are the same or different from each other,
R¹⁰ and R¹¹ each independently are an unsubstituted or substituted C1-6 alkyl group, an unsubstituted or substituted C3-8 cycloalkyl group, an unsubstituted or substituted C6-10 aryl group, an unsubstituted or substituted three- to six-membered heterocyclyl group, an unsubstituted or substituted C1-6 alkoxy group, an unsubstituted or substituted C6-10 aryloxy group, an unsubstituted or substituted heteroaryloxy group, a halogeno group, an unsubstituted or substituted C1-6 alkylthio group, an unsubstituted or substituted C6-10 arylthio group, an unsubstituted or substituted heteroarylthio group, an unsubstituted or substituted C1-6 alkylsulfinyl group, an unsubstituted or substituted C6-10 arylsulfinyl group, an unsubstituted or substituted heteroarylsulfinyl group, an unsubstituted or substituted C1-6 alkylsulfonyl group, an unsubstituted or substituted C6-10 arylsulfonyl group, an unsubstituted or substituted heteroarylsulfonyl group, a cyano group, a nitro group, or a group of formula (III), 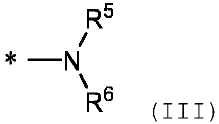
(in formula (III), R⁵ and R⁶ each independently are a hydrogen atom, an unsubstituted or substituted C1-6 alkyl group, an unsubstituted or substituted C6-10 aryl group, an unsubstituted or substituted C1-6 alkylcarbonyl group, or an unsubstituted or substituted C6-10 arylcarbonyl group, and * is a bonding site),
R²⁰ is an unsubstituted or substituted C1-6 alkyl group, an unsubstituted or substituted C3-8 cycloalkyl group, an unsubstituted or substituted C6-10 aryl group, an unsubstituted or substituted three- to six-membered heterocyclyl group, an unsubstituted or substituted C1-6 alkoxy group, an unsubstituted or substituted C6-10 aryloxy group, an unsubstituted or substituted heteroaryloxy group, a halogeno group, an unsubstituted or substituted C1-6 alkylthio group, an unsubstituted or substituted C6-10 arylthio group, an unsubstituted or substituted heteroarylthio group, an unsubstituted or substituted C1-6 alkylsulfinyl group, an unsubstituted or substituted C6-10 arylsulfinyl group, an unsubstituted or substituted heteroarylsulfinyl group, an unsubstituted or substituted C1-6 alkylsulfonyl group, an unsubstituted or substituted C6-10 arylsulfonyl group, an unsubstituted or substituted heteroarylsulfonyl group, a cyano group, a nitro group, or a group of formula (IV), 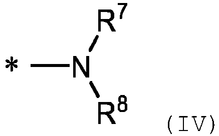
(in formula (IV), R⁷ and R⁸ each independently are a hydrogen atom, an unsubstituted or substituted Cl-6 alkyl group, an unsubstituted or substituted C6-10 aryl group, an unsubstituted or substituted C1-6 alkylcarbonyl group, or an unsubstituted or substituted C6-10 arylcarbonyl group, and * is a bonding site),
q is the number of R²⁰ and is 0, 1, or 2, and when q is 2, R²⁰s are the same or different from each other,
provided that, as substituents on R¹, R², R³, R⁴, R⁵, R⁶, R⁷, R⁸, R¹⁰, R¹¹, and R²⁰, a carboxy group (COOH) and a carboxylate ion group (COO-) are excluded, and in addition, R¹⁰ and R¹¹ are not a methyl group at the same time).

2. The metal-organic framework according to claim 1, wherein the carboxylate ion of formula (I) is a carboxylate ion of formula (V), 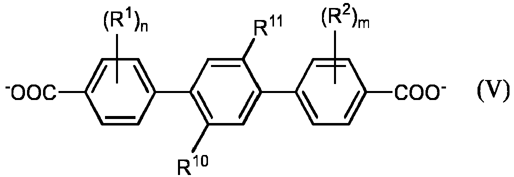 (wherein R¹, R², R¹⁰, R¹¹, n, and m have the same meaning as in formula (I)).

3. The metal-organic framework according to claim 1 or 2, wherein the multivalent metal ion is an ion of at least one metal selected from the group consisting of metals of groups 2 to 13 of the periodic table of elements.

4. The metal-organic framework according to any one of claims 1 to 3, wherein the multivalent metal ion is an ion of at least one metal selected from Zn, Fe, Co, Ni, Cu, Al, Zr, Ti, Cr, and Mg.

5. The metal-organic framework according to any one of claims 1 to 4, further comprising an organic ligand other than the carboxylate ion of formula (I) as a constitution component.

6. A gas storage method comprising a step of bringing a gas into contact with the metal-organic framework according to any one of claims 1 to 5 to adsorb or occlude the gas inside of the metal-organic framework.
